(19) Europäisches Patentamt
European Patent Office
Office européen des brevets

(11) Veröffentlichungsnummer : **0 337 946 B1**

(12) **EUROPÄISCHE PATENTSCHRIFT**

(45) Veröffentlichungstag der Patentschrift :
**28.07.93 Patentblatt 93/30**

(51) Int. Cl.$^5$ : **C07D 239/42,** A01N 43/54

(21) Anmeldenummer : **89810254.6**

(22) Anmeldetag : **04.04.89**

(54) **Harnstoffe.**

(30) Priorität : **12.04.88 CH 1335/88**

(43) Veröffentlichungstag der Anmeldung :
**18.10.89 Patentblatt 89/42**

(45) Bekanntmachung des Hinweises auf die Patenterteilung :
**28.07.93 Patentblatt 93/30**

(84) Benannte Vertragsstaaten :
**AT BE CH DE ES FR GB GR IT LI LU NL SE**

(56) Entgegenhaltungen :
**EP-A- 0 013 143**
**EP-A- 0 135 472**
**EP-A- 0 172 786**
**EP-A- 0 243 136**
**EP-A- 0 264 348**
**DD-A- 151 404**

(73) Patentinhaber : **CIBA-GEIGY AG**
**Klybeckstrasse 141**
**CH-4002 Basel (CH)**

(72) Erfinder : **Rempfler, Hermann, Dr.**
**Brücklismattstrasse 16**
**CH-4107 Ettigen (CH)**

EP 0 337 946 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue N-(2-Nitrophenyl)-N-pyrimidin-2-yl -harnstoffe mit herbizider und pflanzenwuchsregulierender Wirkung, agrochemische Mittel, welche diese Substanzen als Wirkstoffe enthalten, die Verwendung der neuen Harnstoffe zur Bekämpfung von Unkräutern oder zur Regulierung des Pflanzenwuchses sowie verfahren zur Herstellung der neuen Verbindungen. Ferner betrifft die Erfindung auch neue Zwischenprodukte und verfahren zu deren Herstellung.

Aus der Patentschrift DD-151 404 und der europäischen Patentanmeldung EP-A-0 172 786 sind (Pyrimidin-2-yl)-2-nitroaniline bekannt geworden. Diese Verbindungen sind fungizid wirksam. Demgegenüber wurde überraschenderweise gefunden, dass N-Pyrimidin-2-yl-N-2-nitrophenylharnstoffe herbizide bzw. pflanzenwachstumsregulatorische Wirkung haben. Aus der nicht vorpublizierten EP-A-0 264 348 sind herbizid wirksame N-(2-Nitrophenyl)-N-pyrimidin-2-yl-harnstoffe bekannt geworden, von denen sich die erfindungsgemässen Harnstoffe durch die Auswahl der Substituenten am Phenyl- wie am Pyrimidinyl-Ring unterscheiden.

Die Erfindung betrifft Harnstoffe der Formel I

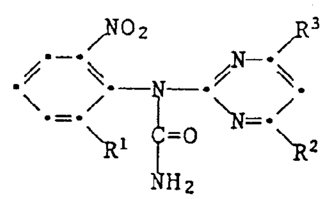

(I),

worin

$R^1$ Wasserstoff, Methyl, Fluor, Chlor oder Brom,

$R^2$ $C_1$-$C_2$-Halogenalkyl mit mindestens 2 Fluoratomen und

$R^3$ $C_1$-$C_4$-Alkyl

bedeutet,

mit der Massgabe, dass $R^2$ nicht Trifluormethyl bedeutet, wenn $R^3$ für Methyl steht, sowie die Salze und Additionsverbindungen der Harnstoffe der Formel I mit Säuren, Basen oder Komplexbildnern.

Im Rahmen der hier offenbarten Erfindung umfassen die angegebenen generischen Begriffe beispielsweise die folgenden spezifischen Einzelsubstituenten, wobei diese Aufzählung keine Einschränkung der Erfindung darstellt:

$C_1$-$C_4$-Alkyl ist Methyl, Ethyl, n-Propyl, iso-Propyl, n-Butyl, sec.-Butyl, iso-Butyl und tert-Butyl, bevorzugt ist Methyl, Ethyl, n-Propyl, iso-Propyl, sec-Butyl und iso-Butyl.

Haloalkylreste sind insbesondere Difluorchlormethyl, Difluormethyl, Trifluormethyl, 1,1-Difluor-2,2,2-trichlormethyl, 1,1-Dichlor-2,2,2-trifluormethyl, Pentafluorethyl, 1,1,2,2-Tetrafluor-2-chlorethyl, 1,1,2,2-Tetrafluorethyl, 1,1,2-Trifluorethyl, 1,2,2,2-Tetrafluorethyl und 1,2,2-Trifluorethyl.

Bevorzugt sind Verbindungen der Formel I, worin

$R^1$ Wasserstoff, Methyl, Fluor, Chlor oder Brom,

$R^2$ Chlordifluormethyl, Difluormethyl, 1,1-Difluor-2,2,2-trichlorethyl, 1,1-Dichlor-2,2,2-trifluorethyl, Pentafluorethyl, 1,1,2,2-Tetrafluor-2-chlorethyl oder Trifluormethyl und

$R^3$ $C_1$-$C_4$-Alkyl bedeutet.

Besonders bevorzugt sind Verbindungen der Formel I,

worin

$R^1$ Wasserstoff, Methyl, Fluor, Chlor oder Brom,

$R^2$ Chlordifluormethyl, Difluormethyl, 1,1-Difluor-2,2,2-trichlorethyl, 1,1-Dichlor-2,2,2-trifluorethyl, Pentafluorethyl, 1,1,2,2-Tetrafluor-2-chlorethyl oder Trifluormethyl und

$R^3$ Methyl, Ethyl, n-Propyl, iso-Propyl, sec-Butyl oder iso-Butyl bedeutet.

Hervorzuheben sind die Verbindungen der Formel I, worin

$R^1$ Wasserstoff oder Methyl,

$R^2$ Trifluormethyl und

$R^3$ Methyl, Ethyl oder Isopropyl bedeutet.

Als Einzelverbindungen zu nennen sind 2-[N-Carbamyl-N-(6-methyl-2-nitrophenyl)-amino]-4-trifluormethyl-6-ethyl-pyrimidin und 2-[N-Carbamyl-N-(2-nitrophenyl)-amino]-4-trifluormethyl-6-ethyl-pyrimidin.

Die Verbindungen der Formel I können hergestellt werden, dadurch dass man

a) ein Anilin der Formel II mit Phosgen zu einem Carbaminchlorid der Formel III umsetzt und dieses in einer zweiten Stufe mit $NH_3$ zu

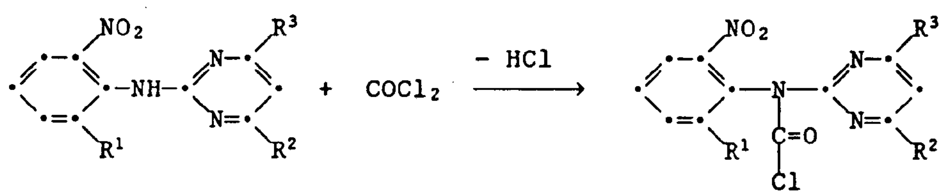


(II) (III)

$$III + NH_3 \xrightarrow{-\,HCl} I$$

einem Harnstoff der Formel I umsetzt oder

b) ein Anilin der Formel II mit Halogensulfonylisocyanat zu einem Halogensulfonylharnstoff der Formel IV umsetzt

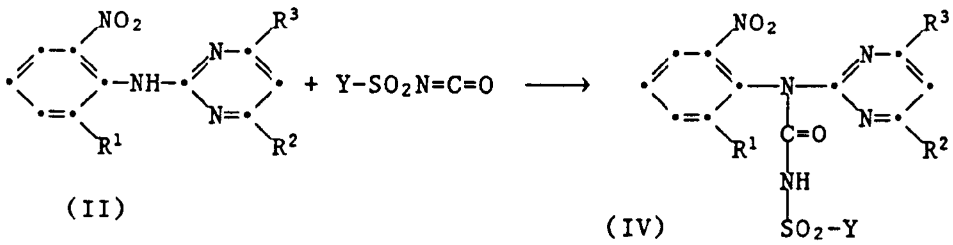


(II) (IV)

$$IV + 2H_2O \xrightarrow[-\,SO_4H_2]{-\,HY} I$$

und diesen in einer zweiten Stufe oder direkt zu einer Verbindung der Formel I hydrolisiert, wobei Y für eine unter den Reaktionsbedingungen abspaltbare Gruppe wie Halogen, vorzugsweise Chlor, steht oder

c) einen Sulfonylharnstoff der Formel V unter Einwirkung einer wässrigen Base zu einem Harnstoff der Formel I umlagert

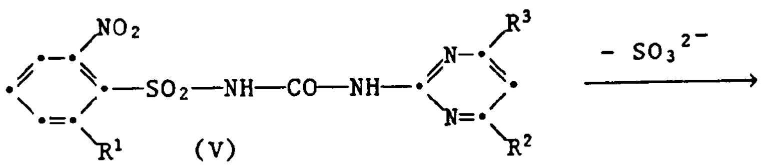


(V) (I),

wobei als wässrige Basen vorzugsweise NaOH/Wasser oder KOH/Wasser verwendet werden.

Die unter Halogenwasserstoffabspaltung bzw. HY-Eliminierung verlaufenden Umsetzungen II → III, III → I und IV → I werden vorzugsweise unter Verwendung säurebindender Mittel (Basen) durchgeführt.

Als solche kommen organische oder anorganische Basen in Betracht, z.B. tertiäre Amine wie Trialkylamine (Trimethylamin, Triethylamin, Tripropylamin usw.), Pyridine (Pyridin, 4-Dimethylaminopyridin, 4-Pyrrolidylaminopyridin usw.), Alkoholate wie z.B. Kalium-tert.-butylat, Natriummethylat oder -ethylat. Die zuvor genannten Reaktionen wie auch die Umsetzung V → I, können auch unter Phasentransferbedingungen mit Basen nach an sich bekannten Verfahren durchgeführt werden (Lit. Dehmlow & Dehmlow, Phase Transfer Catalysis Verlag Chemie, Weinheim, 1983).

Bei den Verfahrensvarianten a) und b) können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Die 2-Nitroaniline der Formel II sind ebenso wie die neuen Carbamoylchloride der Formel III und die neuen Harnstoffe der Formel IV wertvolle Zwischenverbindungen für die Synthese der herbizid wirksamen Harnstoffe I.

Neu sind die Verbindungen der Formel II

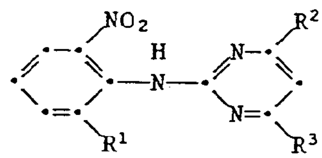

(II),

worin
$R^1$ Wasserstoff, Methyl, Fluor, Chlor oder Brom,
$R^2$ $C_1$-$C_2$-Halogenalkyl mit mindestens 2 Fluoratomen und
$R^3$ $C_1$-$C_4$-Alkyl
bedeutet,
mit der Massgabe, dass $R^2$ nicht Trifluormethyl bedeutet, wenn $R^3$ für Methyl steht.

Neu sind auch die Carbamoylchloride der Formel III

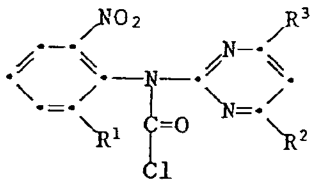

(III),

worin
$R^1$ Wasserstoff, Methyl, Fluor, Chlor oder Brom,
$R^2$ $C_1$-$C_2$-Halogenalkyl mit mindestens 2 Fluoratomen und
$R^3$ $C_1$-$C_4$-Alkyl
bedeutet,
mit der Massgabe, dass $R^2$ nicht Trifluormethyl bedeutet, wenn $R^3$ für Methyl steht.

Die Erfindung betrifft ausserdem die Harnstoffe der Formel IV

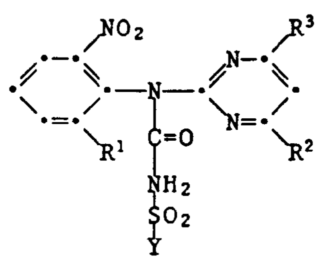

(IV),

worin
Y Halogen,
$R^1$ Wasserstoff, Methyl, Fluor, Chlor oder Brom,
$R^2$ $C_1$-$C_2$-Halogenalkyl mit mindestens 2 Fluoratomen und
$R^3$ $C_1$-$C_4$-Alkyl
bedeutet,
mit der Massgabe, dass $R^2$ nicht Trifluormethyl bedeutet, wenn $R^3$ für Methyl steht.

Die Verbindungen der Formel III und IV sind Zwischenprodukte der Verfahren a) und b) und können wie

dort beschrieben aus den entsprechenden 2-Nitroanilinen der Formel II hergestellt werden.

Die neuen 2-Nitroaniline der Formel II sind analog literaturbekannter Verfahren herstellbar z.B. durch:

d) Umsetzung von Guanidinen der Formel VI mit 1,3-Dicarbonylverbindungen der Formel VII

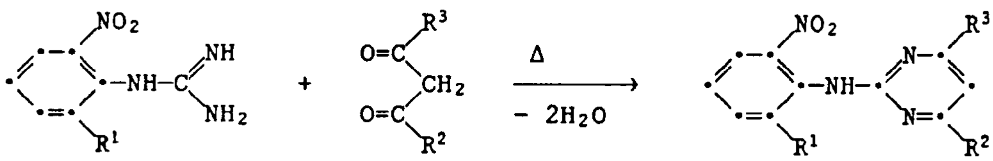


(VI) (VII) (II)

wobei die Kondensationsreaktion gewünschtenfalls in Gegenwart wasser-bindender Mittel durchgeführt wird (Lit: D.J. Brown in "The Chemistry of Heterocyclic Compounds" Bd. VI 1962, Interscience Publ. New York) oder

e) Umsetzung eines Halogenbenzols der Formel VIII mit einem 2-Aminopyrimidin der Formel IX

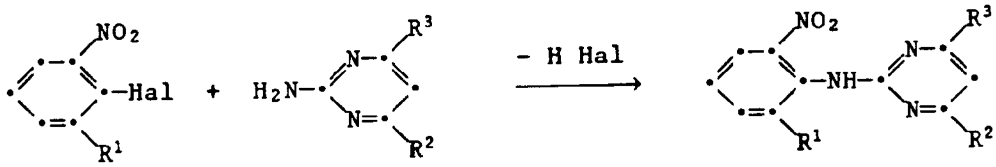


(VIII) (IX) (II)

analog zu EP-A-0 172 786 oder

f) Abbau eines Sulfonylharnstoffes der Formel V unter Einwirkung einer wässrigen Base

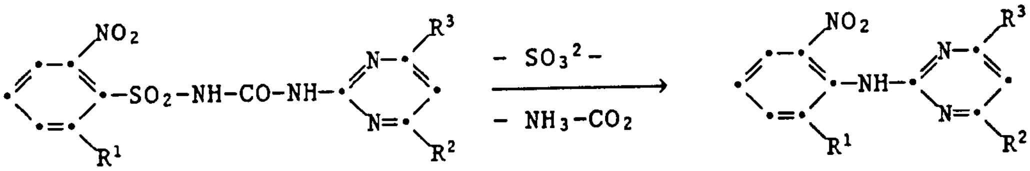


(V) (II)

bei erhöhter Temperatur oder

g) Umsetzung eines Anilins der Formel XIV mit einem Pyrimidin der Formel XII, worin die Reste $R^2$ und $R^3$ wie zuvor definiert sind und X für eine nucleofage Gruppe, wie Halogen, $C_1$-$C_4$-Alkylsulfonyl oder Phenylsulfonyl steht

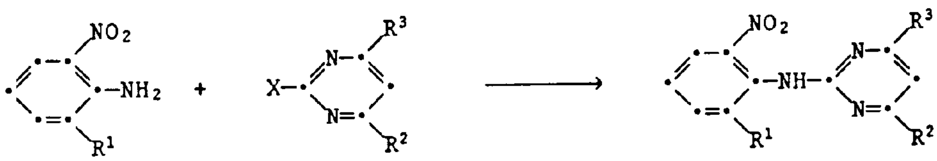

(XIV) (XII) (II)

Die Sulfonylharnstoffe V sind analog zu literaturbekannten Verfahren erhältlich in denen man

h) ein Sulfonamid X, worin $R^1$ wie zuvor definiert ist, mit Phosgen zu einem Isocyanat XI umsetzt und dieses anschliessend mit einem 2-Aminopyrimidin IX zu V reagieren lässt:

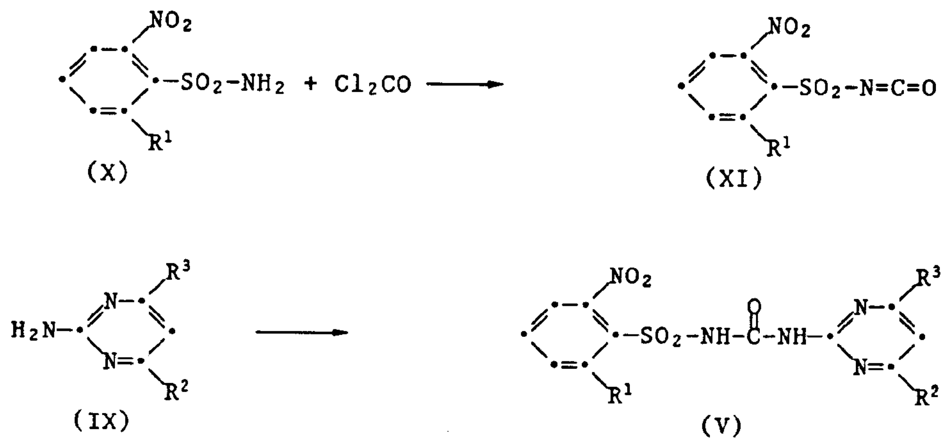

(X) (XI)

(IX) (V)

oder

i) ein 2-Aminopyrimidin IX, worin $R^2$ und $R^3$ wie zuvor definiert sind, mit Phosgen zu einem Isocyanat XIII umsetzt und dieses anschliessend mit einem Sulfonamid X, worin $R^1$ wie zuvor definiert ist, zu V reagieren lässt:

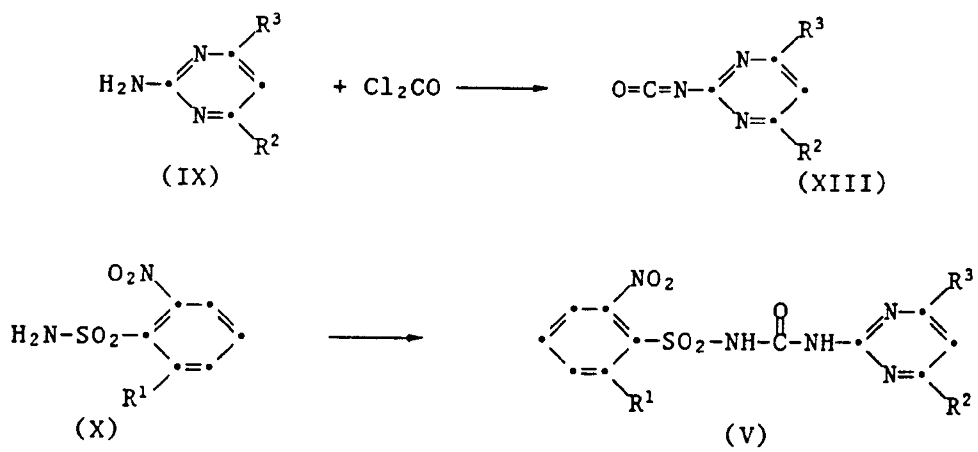

(IX) (XIII)

(X) (V)

Die Guanidine VI sind analog zu literaturbekannten Verfahren darstellbar, indem man

j) ein Anilin XIV, worin $R^1$ wie zuvor definiert ist, mit Cyanamid umsetzt:

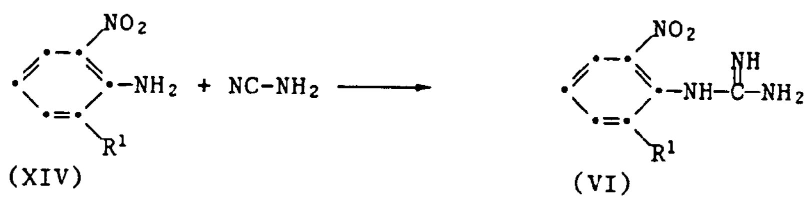

(XIV) (VI)

oder

k) einen Thioharnstoff ($R^4$=H) oder einen Isothioharnstoff ($R^4$=$C_1$-$C_4$-Alkyl) der Formel XV, worin $R^1$ wie zuvor definiert ist und $R^4$ Wasserstoff oder $C_1$-$C_4$-Alkyl bedeutet, mit Ammoniak zum Guanidin VI umsetzt:

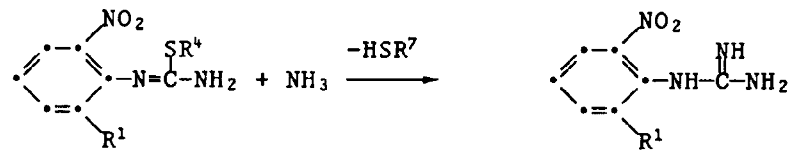

(XV) (VI)

(Literatur: Verfahren j): Houben Weyl, Methoden der Org. Chemie, Thieme, Stuttgart, Bd. VIII S. 98, 180; Verfahren k): Houben Weyl, Methoden der Org. Chemie, Thieme, Stuttgart, Bd. VIII S. 183).

Die β-Diketone VII sind ebenfalls analog zu literaurbekannten Verfahren herstellbar, in dem man l) ein Keton XVI, worin $R^2$ wie zuvor definiert ist, mit einer Verbindung der Formel XVII, worin $R^3$ wie zuvor definiert ist und Z für eine unter den Reaktionsbedingungen der Claisen-Kondensation abspaltbare Gruppe, wie $C_1$-$C_4$-Alkoxy, Phenoxy, Benzyloxy oder Halogen steht, unter den Reaktionsbedingungen einer Claisen Kondensation umsetzt,

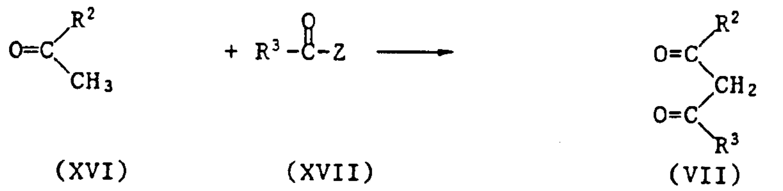

(XVI) (XVII) (VII)

oder

m) ein Keton XIX, worin $R^3$ wie zuvor definiert ist, mit einer Verbindung der Formel XVIII, worin $R^2$ wie zuvor definiert ist und Z für eine unter den Reaktionsbedingungen der Claisen-Kondensation abspaltbare Gruppe, wie $C_1$-$C_4$-Alkoxy, Phenoxy, Benzyloxy oder Halogen steht, unter den Reaktionsbedingungen einer Claisen Kondensation umsetzt,

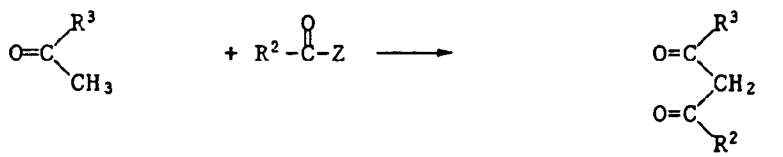

(XVI) (XVII) (VII)

(Lit.: C. Ferry Reaktionen der organischen Synthese, Thieme Stuttgart, 1978 S. 312 sowie dort zitierte Literatur).

Die 2-Aminopyrimidine IX, die 2-Isocyanopyrimidine XIII, die 2-Halogenpyrimidine XII' (mit X=Halogen), die 2-Mercaptopyrimidine XII" (mit X=SH), die 2-Alkylsulfonyl- oder 2-Phenylsulfonylpyrimidine XII"", und die 2-Alkylthiopyrimidine XII''' (mit X=$C_1$-$C_4$-Alkylthio oder Phenylthio) in denen die Reste $R^2$ und $R^3$ wie zuvor definiert sind, sind überwiegend neu.

Zur Herstellung der Verbindungen der Formel XIII, XII', XII" und XII''' stehen dem Fachmann zahlreiche Synthesewege zur Verfügung. Diese sind dem Chemiker allgemein bekannt und in den einschlägigen Handbüchern umfassend beschrieben.

Nachstehendes Syntheseschema (Schema I) zeigt einen Ausschnitt der Möglichkeiten zur Herstellung dieser Verbindungen, wobei jeweils von dem β-Diketon VII ausgegangen wird:

Schema I

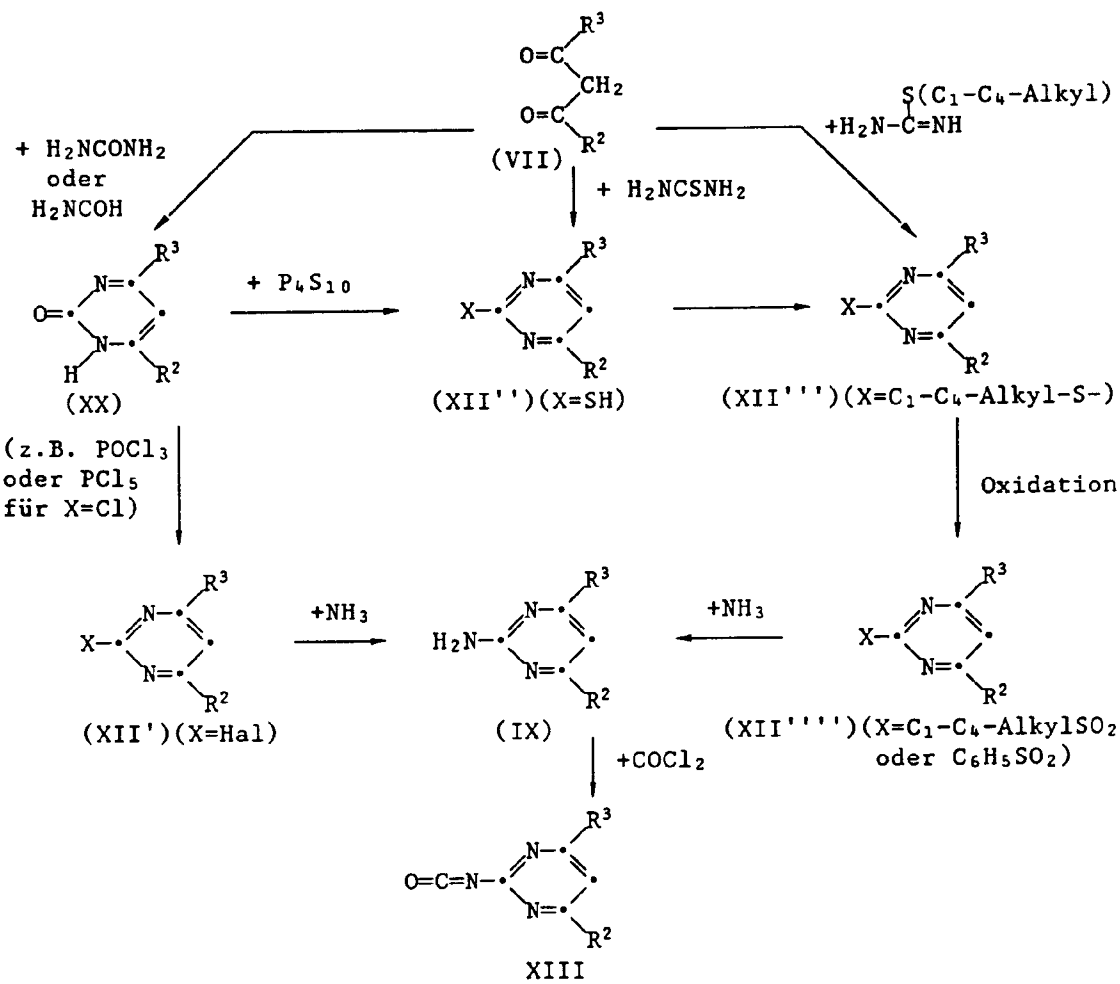


Die Pyridinone XX, worin die Reste $R^2$ und $R^3$ wie zuvor definiert sind, sind ebenfalls neu.

Des weiteren betrifft die Erfindung herbizide und pflanzenwuchsregulatorische Mittel enthaltend eine Verbindung der Formel I zusammen mit geeigneten Hilfs- und/oder Trägerstoffen.

Die Wirkstoffe der Formel I werden in der Regel mit Aufwandmengen von 0,005 bis 5 kg/ha insbesondere 0,1 bis 3 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und herbizide Eigenschaften aus, die sie ausgezeichnet zum Einsatz in Kulturen von Nutzpflanzen, insbesondere in Getreide, Baumwolle, Soja, Sonnenblumen, Raps, Mais und Reis befähigen.

Die Verbindungen der Formel I haben ausserdem pflanzenwuchsregulatorische Eigenschaften. Es werden sowohl Monokotyledonen als auch Dikotyledonen in ihrem Wachstum beeinflusst.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumsregulatoren beruht darauf, dass die Nährstoffe in stärkerem Masse der Blüten- und Fruchtbildung zugute kommen, während das vegetative Wachstum eingeschränkt wird.

In geeigneten Aufwandmengen hemmen die Verbindungen der Formel I den Neuzuwachs von Gräsern. Dies erlaubt es in Rasenkulturen (Parks, Gärten etc.) die Zahl der erforderlichen Schnitte zu vermindern, beziehungsweise die Zeiträume zwischen den einzelnen Schnitten zu verlängern. In besonders vorteilhafter Weise können hierzu Granulatformulierungen der Wirkstoffe der Formel I verwendet werden. Das Granulat kann

entweder den Wirkstoff allein, neben den üblichen Hilfs- und Trägerstoffen, enthalten, oder der Wirkstoff wird zusammen mit einem Mineraldünger, und/oder gegebenenfalls weiteren Wirkstoffen zur Bekämpfung von Moos oder anderen in Rasenkulturen unerwünschten Pflanzenwuchses, als Granulat formuliert. Die Anwendung als Streugranulat erlaubt es, mit Hilfe der bei Rasenkulturen üblichen Bearbeitungsgeräte, während längerer Zeit den Neuzuwachs der Gräser zu hemmen. Das Granulat kann dabei in an sich bekannter Weise hergestellt werden, es weist vorzugsweise eine Korngrösse von 0,1 bis 2,0 mm insbesondere von 0,25 bis 1,0 mm auf.

Als pflanzenwuchsregulatorisch wirksame Verbindungen sind insbesondere zu nennen 2-[N-Carbamyl-N-(6-methyl-2-nitrophenyl)-amino]-4-trifluormethyl-6-ethyl-pyrimidin und 2-[N-Carbamyl-N-(2-nitrophenyl)-amino]-4-trifluormethyl-6-ethyl-pyrimidin.

Bei grösseren Aufwandmengen werden Unkräuter und Gräser in ihrer Entwicklung so geschädigt, dass sie absterben.

In besonders vorteilhafter Weise können die wuchsregulatorischen Verbindungen der Formel I zur Wuchsregulation von Untersaaten in Maiskulturen verwendet werden.

Als Untersaaten in Maiskulturen sind prinzipiell diejenigen Pflanzen geeignet, die den Boden zwischen den einzelnen Maispflanzen bedecken und so in erster Linie der Bodenerosion in Maiskulturen entgegenwirken.

Geeignete Pflanzen für die Untersaat sind unter anderem Raps, Klee, Gräser oder Leguminosen.

Die Erfindung betrifft auch herbizide und pflanzenwachstumsregulierende Mittel, welche einen Wirkstoff der Formel I enthalten, sowie Verfahren zur pre- und post-emergenten Unkrautbekämpfung und zur Beinflussung des Pflanzenwuchses von monokotylen und dikotylen Pflanzen, insbesondere Gräsern, tropischen Bodenbedeckern und Geiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

So können die Aktivsubstanzen der Formel I auch auf mineralische Dünger aufgebracht (aufgebeizt) werden. Das so erhältliche Mittel ist in vorteilhafter Weise als Wuchsregulator bei Gräsern geeignet.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkalioder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäure von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Klebstoffe sind insbesondere diejenigen Hilfsstoffe, welche beim Granulieren den Zusammenhalt des Trägermaterials, der Hilfsstoffe und der Wirkstoffe bewirken, wie Gummi arabicum oder Carboxymethylcellulose.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979.

Dr. Helmut Stache "Tensid Taschenbuch"
Carl Hanser Verlag, München/Wien 1981.

Die Zubereitungen enthalten in der Regel 0,1 bis 95%, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

<u>Emulgierbare Konzentrate:</u>

| | |
|---|---|
| Wirkstoff der Formel I: | 1 bis 20 % bevorzugt 5 bis 10 % |
| oberflächenaktives Mittel: | 5 bis 30 %,vorzugsweise 10 bis 20 % |
| flüssiges Trägermittel: | 50 bis 94 %,vorzugsweise 70 bis 85 %. |

<u>Stäube:</u>

| | |
|---|---|
| Wirkstoff der Formel I: | 0,1 bis 10 %, vorzugsweise 0,1 bis 1 % |
| festes Trägermittel: | 99,9 bis 90 %, vorzugsweise 99,9 bis 99 %. |

Suspensions-Konzentrate:

| | |
|---|---|
| Wirkstoff der Formel I: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

Benetzbare Pulver:

| | |
|---|---|
| Wirkstoff der Formel I: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 %. |

Granulate:

| | |
|---|---|
| Wirkstoff der Formel I: | 0,5 bis 30 %, vorzugsweise 3 bis 15 % |
| festes Trägermittel: | 99,5 bis 70 %, vorzugsweise 97 bis 85 %. |

Streugranulat

| | |
|---|---|
| Wirkstoff der Formel I | 0,01 bis 30 %, vorzugsweise 0,05 bis 15 % |
| Klebstoff | 0,05 bis 5 %, vorzugsweise 0,1 bis 2 % |
| oberflächenaktives Mittel | 0,5 bis 20 %, vorzugsweise 1 bis 15 % |
| festes Trägermaterial | 99,44 bis 45 %, vorzugsweise 95 bis 65 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

Die - unkorrigierten - Schmelzpunkte in den nachfolgenden Herstellungsbeispielen sind in °C angegeben.

H.1.1. Synthese von N-2-Nitrophenyl-N-(4-methyl-6-pentafluorethylpyrimidin-2-yl)-harnstoff

104,1 g (0,3 Mol) 2-(2-Nitrophenylamino)-4-methyl-6-pentafluorethylpyrimidin werden bei 60°C in 2,2 l Essigsäureethylester gelöst, danach wird auf 5°C gekühlt und mit 56,6 g (0,4 Mol) Chlorsulfonylisocyanat versetzt und 15 Min. bei 5°C gerührt. Danach werden 300 ml kaltes Wasser zugegossen, die organische Phase wird abgetrennt, mit Natriumsulfat getrocknet und eingeengt. Der feste Rückstand wird mit wenig Essigsäureethylester verrieben.

Man isoliert 73,9 g (63,2 %) der Titelverbindung der Formel

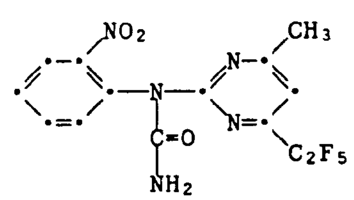

(Verb. No 1.025) als Kristalle vom Smp. 156°C.

H.1.2. Synthese von N-2-Nitrophenyl-N-(4-methyl-6-pentafluorethylpyrimidin-2-yl)-harnstoff

21,5 g (0,05 Mol) N-(2-Nitrophenylsulfonyl)-N'-(4-methyl-6-pentafluorethyl-pyrimidin-2-yl)-harnstoff werden in 100 ml Wasser und 200 ml Chloroform suspendiert. Dazu tropft man bei 25° innert 4 Std. eine Lösung von 3.2 g Natriumhydroxid in 350 ml Wasser. Die 2-phasige Lösung wird 16 Std. gerührt. Dann wird die $CHCl_3$-Phase abgetrennt, mit Natriumsulfat getrocknet und eingedampft. Der Rückstand wird aus Chloroform umkristallisiert.

Man isoliert 4,0 g (20.7 %) der Titelverbindung der Formel

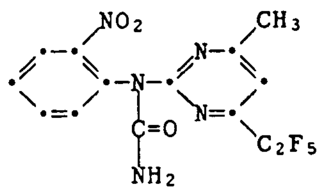

(Verbindung No. 1.025) als Kristalle vom Smp. 156°C.

Analog zu den Herstellungsbeispielen H.1.1. und H.1.2. können die nachstehend in Tabelle 1 aufgeführten Verbindungen der Formel I

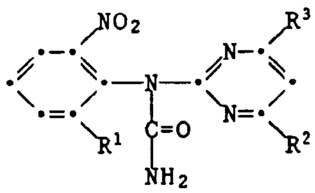

(I)

hergestellt werden:

Tabelle I

| Verb. No. | $R^1$ | $R^2$ | $R^3$ | phys. Daten |
|---|---|---|---|---|
| 1.001 | H | $CF_2Cl$ | $CH_3$ | Fp. 156°C |
| 1.002 | H | $CF_2Cl$ | $C_2H_5$ | |
| 1.003 | H | $CF_2Cl$ | $n-C_3H_7$ | |
| 1.004 | H | $CF_2Cl$ | $i-C_3H_7$ | Fp. 149-150°C |
| 1.005 | H | $CF_2Cl$ | $sec-C_4H_9$ | |
| 1.006 | H | $CF_2Cl$ | $i-C_4H_9$ | |
| 1.007 | H | $CHF_2$ | $CH_3$ | Fp. 155-156°C |
| 1.008 | H | $CHF_2$ | $C_2H_5$ | |
| 1.009 | H | $CHF_2$ | $n-C_3H_7$ | |
| 1.010 | H | $CHF_2$ | $i-C_3H_7$ | |
| 1.011 | H | $CHF_2$ | $sec-C_4H_9$ | |
| 1.012 | H | $CHF_2$ | $i-C_4H_9$ | |
| 1.013 | H | $CF_2CCl_3$ | $CH_3$ | |
| 1.014 | H | $CF_2CCl_3$ | $C_2H_5$ | |
| 1.015 | H | $CF_2CCl_3$ | $n-C_3H_7$ | |
| 1.016 | H | $CF_2CCl_3$ | $i-C_3H_7$ | |
| 1.017 | H | $CF_2CCl_3$ | $sec-C_4H_9$ | |
| 1.018 | H | $CF_2CCl_3$ | $i-C_4H_9$ | |
| 1.019 | H | $CCl_2CF_3$ | $CH_3$ | |
| 1.020 | H | $CCl_2CF_3$ | $C_2H_5$ | |
| 1.021 | H | $CCl_2CF_3$ | $n-C_3H_7$ | |
| 1.022 | H | $CCl_2CF_3$ | $i-C_3H_7$ | |
| 1.023 | H | $CCl_2CF_3$ | $sec-C_4H_9$ | |
| 1.024 | H | $CCl_2CF_3$ | $i-C_4H_9$ | |
| 1.025 | H | $C_2F_5$ | $CH_3$ | Fp. 156°C |
| 1.026 | H | $C_2F_5$ | $C_2H_5$ | Fp. 156-157°C |
| 1.027 | H | $C_2F_5$ | $n-C_3H_7$ | |
| 1.028 | H | $C_2F_5$ | $i-C_3H_7$ | Fp. 152-153° |
| 1.029 | H | $C_2F_5$ | $sec-C_4H_9$ | |
| 1.030 | H | $C_2F_5$ | $i-C_4H_9$ | |
| 1.031 | H | $CF_2-CF_2Cl$ | $CH_3$ | |
| 1.032 | H | $CF_2-CF_2Cl$ | $C_2H_5$ | |
| 1.033 | H | $CF_2-CF_2Cl$ | $n-C_3H_7$ | |
| 1.034 | H | $CF_2-CF_2Cl$ | $i-C_3H_7$ | |
| 1.035 | H | $CF_2-CF_2Cl$ | $sec-C_4H_9$ | |
| 1.036 | H | $CF_2-CF_2Cl$ | $i-C_4H_9$ | |
| 1.037 | H | $CF_3$ | $C_2H_5$ | Fp. 137-138°C |
| 1.038 | H | $CF_3$ | $i\ C_3H_7$ | Fp. 150-151°C |
| 1.039 | H | $CF_3$ | $sec-C_4H_9$ | Fp. 141-142°C |
| 1.040 | H | $CF_3$ | $i-C_4H_9$ | |

Tabelle I (Fortsetzung)

| Verb. No. | $R^1$ | $R^2$ | $R^3$ | phys. Daten |
|---|---|---|---|---|
| 1.041 | $CH_3$ | $CF_2Cl$ | $CH_3$ | Fp. 178-179°C |
| 1.042 | $CH_3$ | $CF_2Cl$ | $C_2H_5$ | |
| 1.043 | $CH_3$ | $CF_2Cl$ | n-$C_3H_7$ | |
| 1.044 | $CH_3$ | $CF_2Cl$ | i-$C_3H_7$ | Fp. 153-154°C |
| 1.045 | $CH_3$ | $CF_2Cl$ | sec-$C_4H_9$ | |
| 1.046 | $CH_3$ | $CF_2Cl$ | i-$C_4H_9$ | |
| 1.047 | $CH_3$ | $CHF_2$ | $CH_3$ | Fp. 158-160°C |
| 1.048 | $CH_3$ | $CHF_2$ | $C_2H_5$ | |
| 1.049 | $CH_3$ | $CHF_2$ | n-$C_3H_7$ | |
| 1.050 | $CH_3$ | $CHF_2$ | i-$C_3H_7$ | |
| 1.051 | $CH_3$ | $CHF_2$ | sec-$C_4H_9$ | |
| 1.052 | $CH_3$ | $CHF_2$ | i-$C_4H_9$ | |
| 1.053 | $CH_3$ | $CF_2CCl_3$ | $CH_3$ | |
| 1.054 | $CH_3$ | $CF_2CCl_3$ | $C_2H_5$ | |
| 1.055 | $CH_3$ | $CF_2CCl_3$ | n-$C_3H_7$ | |
| 1.056 | $CH_3$ | $CF_2CCl_3$ | i-$C_3H_7$ | |
| 1.057 | $CH_3$ | $CF_2CCl_3$ | sec-$C_4H_9$ | |
| 1.058 | $CH_3$ | $CF_2CCl_3$ | i-$C_4H_9$ | |
| 1.059 | $CH_3$ | $CCl_2CF_3$ | $CH_3$ | |
| 1.060 | $CH_3$ | $CCl_2CF_3$ | $C_2H_5$ | |
| 1.061 | $CH_3$ | $CCl_2CF_3$ | n-$C_3H_7$ | |
| 1.062 | $CH_3$ | $CCl_2CF_3$ | i-$C_3H_7$ | |
| 1.063 | $CH_3$ | $CCl_2CF_3$ | sec-$C_4H_9$ | |
| 1.064 | $CH_3$ | $CCl_2CF_3$ | i-$C_4H_9$ | |
| 1.065 | $CH_3$ | $C_2F_5$ | $CH_3$ | |
| 1.066 | $CH_3$ | $C_2F_5$ | $C_2H_5$ | Fp. 135-136°C |
| 1.067 | $CH_3$ | $C_2F_5$ | n-$C_3H_7$ | |
| 1.068 | $CH_3$ | $C_2F_5$ | i-$C_3H_7$ | Fp. 143-144°C |
| 1.069 | $CH_3$ | $C_2F_5$ | sec-$C_4H_9$ | |
| 1.070 | $CH_3$ | $C_2F_5$ | i-$C_4H_9$ | |
| 1.071 | $CH_3$ | $CF_2$-$CF_2Cl$ | $CH_3$ | |
| 1.072 | $CH_3$ | $CF_2$-$CF_2Cl$ | $C_2H_5$ | |
| 1.073 | $CH_3$ | $CF_2$-$CF_2Cl$ | n-$C_3H_7$ | |
| 1.074 | $CH_3$ | $CF_2$-$CF_2Cl$ | i-$C_3H_7$ | |
| 1.075 | $CH_3$ | $CF_2$-$CF_2Cl$ | sec-$C_4H_9$ | |
| 1.076 | $CH_3$ | $CF_2$-$CF_2Cl$ | i-$C_4H_9$ | |
| 1.077 | $CH_3$ | $CF_3$ | $C_2H_5$ | Fp. 149-150°C |
| 1.078 | $CH_3$ | $CF_3$ | i $C_3H_7$ | Fp. 135-138°C |
| 1.079 | $CH_3$ | $CF_3$ | sec-$C_4H_9$ | Fp. 141-142°C |
| 1.080 | $CH_3$ | $CF_3$ | i-$C_4H_9$ | |

Tabelle I (Fortsetzung)

| Verb. No. | $R^1$ | $R^2$ | $R^3$ | phys. Daten |
|---|---|---|---|---|
| 1.081 | F | $CF_2Cl$ | $CH_3$ | |
| 1.082 | F | $CF_2Cl$ | $C_2H_5$ | |
| 1.083 | F | $CF_2Cl$ | $n-C_3H_7$ | |
| 1.084 | F | $CF_2Cl$ | $i-C_3H_7$ | |
| 1.085 | F | $CF_2Cl$ | $sec-C_4H_9$ | |
| 1.086 | F | $CF_2Cl$ | $i-C_4H_9$ | |
| 1.087 | F | $CHF_2$ | $CH_3$ | |
| 1.088 | F | $CHF_2$ | $C_2H_5$ | |
| 1.089 | F | $CHF_2$ | $n-C_3H_7$ | |
| 1.090 | F | $CHF_2$ | $i-C_3H_7$ | |
| 1.091 | F | $CHF_2$ | $sec-C_4H_9$ | |
| 1.092 | F | $CHF_2$ | $i-C_4H_9$ | |
| 1.093 | F | $CF_2CCl_3$ | $CH_3$ | |
| 1.094 | F | $CF_2CCl_3$ | $C_2H_5$ | |
| 1.095 | F | $CF_2CCl_3$ | $n-C_3H_7$ | |
| 1.096 | F | $CF_2CCl_3$ | $i-C_3H_7$ | |
| 1.097 | F | $CF_2CCl_3$ | $sec-C_4H_9$ | |
| 1.098 | F | $CF_2CCl_3$ | $i-C_4H_9$ | |
| 1.099 | F | $CCl_2CF_3$ | $CH_3$ | |
| 1.100 | F | $CCl_2CF_3$ | $C_2H_5$ | |
| 1.101 | F | $CCl_2CF_3$ | $n-C_3H_7$ | |
| 1.102 | F | $CCl_2CF_3$ | $i-C_3H_7$ | |
| 1.103 | F | $CCl_2CF_3$ | $sec-C_4H_9$ | |
| 1.104 | F | $CCl_2CF_3$ | $i-C_4H_9$ | |
| 1.105 | F | $C_2F_5$ | $CH_3$ | |
| 1.106 | F | $C_2F_5$ | $C_2H_5$ | Fp. 143-144°C |
| 1.107 | F | $C_2F_5$ | $n-C_3H_7$ | |
| 1.108 | F | $C_2F_5$ | $i-C_3H_7$ | |
| 1.109 | F | $C_2F_5$ | $sec-C_4H_9$ | |
| 1.110 | F | $C_2F_5$ | $i-C_4H_9$ | |
| 1.111 | F | $CF_2-CF_2Cl$ | $CH_3$ | |
| 1.112 | F | $CF_2-CF_2Cl$ | $C_2H_5$ | |
| 1.113 | F | $CF_2-CF_2Cl$ | $n-C_3H_7$ | |
| 1.114 | F | $CF_2-CF_2Cl$ | $i-C_3H_7$ | |
| 1.115 | F | $CF_2-CF_2Cl$ | $sec-C_4H_9$ | |
| 1.116 | F | $CF_2-CF_2Cl$ | $i-C_4H_9$ | |
| 1.117 | F | $CF_3$ | $C_2H_5$ | Fp. 140-142°C |
| 1.118 | F | $CF_3$ | $i\ C_3H_7$ | Fp. 145-146°C |
| 1.119 | F | $CF_3$ | $sec-C_4H_9$ | |
| 1.120 | F | $CF_3$ | $i-C_4H_9$ | |

Tabelle I (Fortsetzung)

| Verb. No. | $R^1$ | $R^2$ | $R^3$ | phys. Daten |
|---|---|---|---|---|
| 1.121 | Cl | $CF_2Cl$ | $CH_3$ | |
| 1.122 | Cl | $CF_2Cl$ | $C_2H_5$ | |
| 1.123 | Cl | $CF_2Cl$ | $n-C_3H_7$ | |
| 1.124 | Cl | $CF_2Cl$ | $i-C_3H_7$ | |
| 1.125 | Cl | $CF_2Cl$ | $sec-C_4H_9$ | |
| 1.126 | Cl | $CF_2Cl$ | $i-C_4H_9$ | |
| 1.127 | Cl | $CHF_2$ | $CH_3$ | |
| 1.128 | Cl | $CHF_2$ | $C_2H_5$ | |
| 1.129 | Cl | $CHF_2$ | $n-C_3H_7$ | |
| 1.130 | Cl | $CHF_2$ | $i-C_3H_7$ | |
| 1.131 | Cl | $CHF_2$ | $sec-C_4H_9$ | |
| 1.132 | Cl | $CHF_2$ | $i-C_4H_9$ | |
| 1.133 | Cl | $CF_2CCl_3$ | $CH_3$ | |
| 1.134 | Cl | $CF_2CCl_3$ | $C_2H_5$ | |
| 1.135 | Cl | $CF_2CCl_3$ | $n-C_3H_7$ | |
| 1.136 | Cl | $CF_2CCl_3$ | $i-C_3H_7$ | |
| 1.137 | Cl | $CF_2CCl_3$ | $sec-C_4H_9$ | |
| 1.138 | Cl | $CF_2CCl_3$ | $i-C_4H_9$ | |
| 1.139 | Cl | $CCl_2CF_3$ | $CH_3$ | |
| 1.140 | Cl | $CCl_2CF_3$ | $C_2H_5$ | |
| 1.141 | Cl | $CCl_2CF_3$ | $n-C_3H_7$ | |
| 1.142 | Cl | $CCl_2CF_3$ | $i-C_3H_7$ | |
| 1.143 | Cl | $CCl_2CF_3$ | $sec-C_4H_9$ | |
| 1.144 | Cl | $CCl_2CF_3$ | $i-C_4H_9$ | |
| 1.145 | Cl | $C_2F_5$ | $CH_3$ | |
| 1.146 | Cl | $C_2F_5$ | $C_2H_5$ | |
| 1.147 | Cl | $C_2F_5$ | $n-C_3H_7$ | |
| 1.148 | Cl | $C_2F_5$ | $i-C_3H_7$ | |
| 1.149 | Cl | $C_2F_5$ | $sec-C_4H_9$ | |
| 1.150 | Cl | $C_2F_5$ | $i-C_4H_9$ | |
| 1.151 | Cl | $CF_2-CF_2Cl$ | $CH_3$ | |
| 1.152 | Cl | $CF_2-CF_2Cl$ | $C_2H_5$ | |
| 1.153 | Cl | $CF_2-CF_2Cl$ | $n-C_3H_7$ | |
| 1.154 | Cl | $CF_2-CF_2Cl$ | $i-C_3H_7$ | |
| 1.155 | Cl | $CF_2-CF_2Cl$ | $sec-C_4H_9$ | |
| 1.156 | Cl | $CF_2-CF_2Cl$ | $i-C_4H_9$ | |
| 1.157 | Cl | $CF_3$ | $C_2H_5$ | |
| 1.158 | Cl | $CF_3$ | $i\ C_3H_7$ | |
| 1.159 | Cl | $CF_3$ | $sec-C_4H_9$ | |
| 1.160 | Cl | $CF_3$ | $i-C_4H_9$ | |

Tabelle I (Fortsetzung)

| Verb. No. | $R^1$ | $R^2$ | $R^3$ | phys. Daten |
|---|---|---|---|---|
| 1.161 | Br | $CF_2Cl$ | $CH_3$ | |
| 1.162 | Br | $CF_2Cl$ | $C_2H_5$ | |
| 1.163 | Br | $CF_2Cl$ | $n-C_3H_7$ | |
| 1.164 | Br | $CF_2Cl$ | $i-C_3H_7$ | |
| 1.165 | Br | $CF_2Cl$ | $sec-C_4H_9$ | |
| 1.166 | Br | $CF_2Cl$ | $i-C_4H_9$ | |
| 1.167 | Br | $CHF_2$ | $CH_3$ | |
| 1.168 | Br | $CHF_2$ | $C_2H_5$ | |
| 1.169 | Br | $CHF_2$ | $n-C_3H_7$ | |
| 1.170 | Br | $CHF_2$ | $i-C_3H_7$ | |
| 1.171 | Br | $CHF_2$ | $sec-C_4H_9$ | |
| 1.172 | Br | $CHF_2$ | $i-C_4H_9$ | |
| 1.173 | Br | $CF_2CCl_3$ | $CH_3$ | |
| 1.174 | Br | $CF_2CCl_3$ | $C_2H_5$ | |
| 1.175 | Br | $CF_2CCl_3$ | $n-C_3H_7$ | |
| 1.176 | Br | $CF_2CCl_3$ | $i-C_3H_7$ | |
| 1.177 | Br | $CF_2CCl_3$ | $sec-C_4H_9$ | |
| 1.178 | Br | $CF_2CCl_3$ | $i-C_4H_9$ | |
| 1.179 | Br | $CCl_2CF_3$ | $CH_3$ | |
| 1.180 | Br | $CCl_2CF_3$ | $C_2H_5$ | |
| 1.181 | Br | $CCl_2CF_3$ | $n-C_3H_7$ | |
| 1.182 | Br | $CCl_2CF_3$ | $i-C_3H_7$ | |
| 1.183 | Br | $CCl_2CF_3$ | $sec-C_4H_9$ | |
| 1.184 | Br | $CCl_2CF_3$ | $i-C_4H_9$ | |
| 1.185 | Br | $C_2F_5$ | $CH_3$ | |
| 1.186 | Br | $C_2F_5$ | $C_2H_5$ | |
| 1.187 | Br | $C_2F_5$ | $n-C_3H_7$ | |
| 1.188 | Br | $C_2F_5$ | $i-C_3H_7$ | |
| 1.189 | Br | $C_2F_5$ | $sec-C_4H_9$ | |
| 1.190 | Br | $C_2F_5$ | $i-C_4H_9$ | |
| 1.191 | Br | $CF_2-CF_2Cl$ | $CH_3$ | |
| 1.192 | Br | $CF_2-CF_2Cl$ | $C_2H_5$ | |
| 1.193 | Br | $CF_2-CF_2Cl$ | $n-C_3H_7$ | |
| 1.194 | Br | $CF_2-CF_2Cl$ | $i-C_3H_7$ | |
| 1.195 | Br | $CF_2-CF_2Cl$ | $sec-C_4H_9$ | |
| 1.196 | Br | $CF_2-CF_2Cl$ | $i-C_4H_9$ | |
| 1.197 | Br | $CF_3$ | $C_2H_5$ | |
| 1.198 | Br | $CF_3$ | $i\ C_3H_7$ | |
| 1.199 | Br | $CF_3$ | $sec-C_4H_9$ | |
| 1.200 | Br | $CF_3$ | $i-C_4H_9$ | |
| 1.201 | H | $CF_3$ | $n-C_3H_7$ | Fp. 138-139°C |
| 1.202 | $CH_3$ | $CF_3$ | $n-C_3H_7$ | Fp. 136-137°C |
| 1.203 | F | $CF_3$ | $n-C_3H_7$ | |
| 1.204 | Cl | $CF_3$ | $n-C_3H_7$ | |
| 1.205 | Br | $CF_3$ | $n-C_3H_7$ | |

H.2.1. Synthese von 2-(2-Nitrophenylamino)-4-methyl-6-pentafluorethylpyrimidin

121 g (0,5 Mol) 2-Nitrophenyl-guanidin-carbonat, 140 g 5,5,6,6,6-pentafluorhexan-2,4-dion und 200 ml Diethylenglycoldimethylether werden zunächst auf 65° C und nach Beendigung der $CO_2$-Entwicklung auf 140°C erwärmt. Dabei lässt man das bei der Kondensation freiwerdende Wasser abdestillieren. Nach 3 1/2 Std. lässt man die Suspension abkühlen, gibt Wasser zu und stellt den pH mit konzentrierter Salzsäure auf 4-5. Der gelbe Niederschlag wird abfiltriert, mit Wasser gewaschen und im Vakuum bei 80°C getrocknet.

Man isoliert 110,5 g (63,7 %) der Titelverbindung der Formel

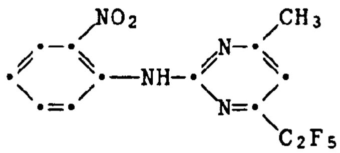

(Verb. No. 2.025) als Kristalle vom Smp. 82-83°C.

Analog zu Herstellungsbeispiel H.2.1. können die in Tabelle 2 aufgeführten Verbindungen der Formel II

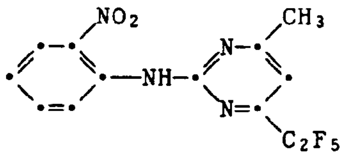

hergestellt werden.

Tabelle 2

| Verb. No. | $R^1$ | $R^2$ | $R^3$ | phys. Daten |
|---|---|---|---|---|
| 2.001 | H | $CF_2Cl$ | $CH_3$ | Fp. 127-128°C |
| 2.002 | H | $CF_2Cl$ | $C_2H_5$ | |
| 2.003 | H | $CF_2Cl$ | $n-C_3H_7$ | |
| 2.004 | H | $CF_2Cl$ | $i-C_3H_7$ | Fp. 52-54°C |
| 2.005 | H | $CF_2Cl$ | $sec-C_4H_9$ | |
| 2.006 | H | $CF_2Cl$ | $i-C_4H_9$ | |
| 2.007 | H | $CHF_2$ | $CH_3$ | Fp. 143-144°C |
| 2.008 | H | $CHF_2$ | $C_2H_5$ | |
| 2.009 | H | $CHF_2$ | $n-C_3H_7$ | |
| 2.010 | H | $CHF_2$ | $i-C_3H_7$ | |
| 2.011 | H | $CHF_2$ | $sec-C_4H_9$ | |
| 2.012 | H | $CHF_2$ | $i-C_4H_9$ | |
| 2.013 | H | $CF_2CCl_3$ | $CH_3$ | |
| 2.014 | H | $CF_2CCl_3$ | $C_2H_5$ | |
| 2.015 | H | $CF_2CCl_3$ | $n-C_3H_7$ | |
| 2.016 | H | $CF_2CCl_3$ | $i-C_3H_7$ | |
| 2.017 | H | $CF_2CCl_3$ | $sec-C_4H_9$ | |
| 2.018 | H | $CF_2CCl_3$ | $i-C_4H_9$ | |
| 2.019 | H | $CCl_2CF_3$ | $CH_3$ | |
| 2.020 | H | $CCl_2CF_3$ | $C_2H_5$ | |
| 2.021 | H | $CCl_2CF_3$ | $n-C_3H_7$ | |
| 2.022 | H | $CCl_2CF_3$ | $i-C_3H_7$ | |
| 2.023 | H | $CCl_2CF_3$ | $sec-C_4H_9$ | |
| 2.024 | H | $CCl_2CF_3$ | $i-C_4H_9$ | |
| 2.025 | H | $C_2F_5$ | $CH_3$ | Fp. 82-83°C |
| 2.026 | H | $C_2F_5$ | $C_2H_5$ | Fp. 82-83°C |
| 2.027 | H | $C_2F_5$ | $n-C_3H_7$ | |
| 2.028 | H | $C_2F_5$ | $i-C_3H_7$ | Fp. 64-65°C |
| 2.029 | H | $C_2F_5$ | $sec-C_4H_9$ | |
| 2.030 | H | $C_2F_5$ | $i-C_4H_9$ | |
| 2.031 | H | $CF_2-CF_2Cl$ | $CH_3$ | |
| 2.032 | H | $CF_2-CF_2Cl$ | $C_2H_5$ | |
| 2.033 | H | $CF_2-CF_2Cl$ | $n-C_3H_7$ | |
| 2.034 | H | $CF_2-CF_2Cl$ | $i-C_3H_7$ | |
| 2.035 | H | $CF_2-CF_2Cl$ | $sec-C_4H_9$ | |
| 2.036 | H | $CF_2-CF_2Cl$ | $i-C_4H_9$ | |
| 2.037 | H | $CF_3$ | $C_2H_5$ | Fp. 71-72°C |
| 2.038 | H | $CF_3$ | $i\ C_3H_7$ | Fp. 72-73°C |
| 2.039 | H | $CF_3$ | $sec-C_4H_9$ | $n_D^{25}$ 1.5730 |
| 2.040 | H | $CF_3$ | $i-C_4H_9$ | |

Tabelle 2 (Fortsetzung)

| Verb. No. | $R^1$ | $R^2$ | $R^3$ | phys. Daten |
|---|---|---|---|---|
| 2.041 | $CH_3$ | $CF_2Cl$ | $CH_3$ | Fp. 96-97°C |
| 2.042 | $CH_3$ | $CF_2Cl$ | $C_2H_5$ | |
| 2.043 | $CH_3$ | $CF_2Cl$ | n-$C_3H_7$ | |
| 2.044 | $CH_3$ | $CF_2Cl$ | i-$C_3H_7$ | Fp. 67-69°C |
| 2.045 | $CH_3$ | $CF_2Cl$ | sec-$C_4H_9$ | |
| 2.046 | $CH_3$ | $CF_2Cl$ | i-$C_4H_9$ | |
| 2.047 | $CH_3$ | $CHF_2$ | $CH_3$ | Fp. 126-127°C |
| 2.048 | $CH_3$ | $CHF_2$ | $C_2H_5$ | |
| 2.049 | $CH_3$ | $CHF_2$ | n-$C_3H_7$ | |
| 2.050 | $CH_3$ | $CHF_2$ | i-$C_3H_7$ | |
| 2.051 | $CH_3$ | $CHF_2$ | sec-$C_4H_9$ | |
| 2.052 | $CH_3$ | $CHF_2$ | i-$C_4H_9$ | |
| 2.053 | $CH_3$ | $CF_2CCl_3$ | $CH_3$ | |
| 2.054 | $CH_3$ | $CF_2CCl_3$ | $C_2H_5$ | |
| 2.055 | $CH_3$ | $CF_2CCl_3$ | n-$C_3H_7$ | |
| 2.056 | $CH_3$ | $CF_2CCl_3$ | i-$C_3H_7$ | |
| 2.057 | $CH_3$ | $CF_2CCl_3$ | sec-$C_4H_9$ | |
| 2.058 | $CH_3$ | $CF_2CCl_3$ | i-$C_4H_9$ | |
| 2.059 | $CH_3$ | $CCl_2CF_3$ | $CH_3$ | |
| 2.060 | $CH_3$ | $CCl_2CF_3$ | $C_2H_5$ | |
| 2.061 | $CH_3$ | $CCl_2CF_3$ | n-$C_3H_7$ | |
| 2.062 | $CH_3$ | $CCl_2CF_3$ | i-$C_3H_7$ | |
| 2.063 | $CH_3$ | $CCl_2CF_3$ | sec-$C_4H_9$ | |
| 2.064 | $CH_3$ | $CCl_2CF_3$ | i-$C_4H_9$ | |
| 2.065 | $CH_3$ | $C_2F_5$ | $CH_3$ | |
| 2.066 | $CH_3$ | $C_2F_5$ | $C_2H_5$ | Fp. 87-88°C |
| 2.067 | $CH_3$ | $C_2F_5$ | n-$C_3H_7$ | |
| 2.068 | $CH_3$ | $C_2F_5$ | i-$C_3H_7$ | Fp. 50-51°C |
| 2.069 | $CH_3$ | $C_2F_5$ | sec-$C_4H_9$ | |
| 2.070 | $CH_3$ | $C_2F_5$ | i-$C_4H_9$ | |
| 2.071 | $CH_3$ | $CF_2$-$CF_2Cl$ | $CH_3$ | |
| 2.072 | $CH_3$ | $CF_2$-$CF_2Cl$ | $C_2H_5$ | |
| 2.073 | $CH_3$ | $CF_2$-$CF_2Cl$ | n-$C_3H_7$ | |
| 2.074 | $CH_3$ | $CF_2$-$CF_2Cl$ | i-$C_3H_7$ | |
| 2.075 | $CH_3$ | $CF_2$-$CF_2Cl$ | sec-$C_4H_9$ | |
| 2.076 | $CH_3$ | $CF_2$-$CF_2Cl$ | i-$C_4H_9$ | |
| 2.077 | $CH_3$ | $CF_3$ | $C_2H_5$ | Fp. 84-85°C |
| 2.078 | $CH_3$ | $CF_3$ | i $C_3H_7$ | $n_D^{30}$: 1,5382 |
| 2.079 | $CH_3$ | $CF_3$ | sec-$C_4H_9$ | $n_D^{25}$: 1,5318 |
| 2.080 | $CH_3$ | $CF_3$ | i-$C_4H_9$ | |

Tabelle 2 (Fortsetzung)

| Verb. No. | $R^1$ | $R^2$ | $R^3$ | phys. Daten |
|---|---|---|---|---|
| 2.081 | F | $CF_2Cl$ | $CH_3$ | |
| 2.082 | F | $CF_2Cl$ | $C_2H_5$ | |
| 2.083 | F | $CF_2Cl$ | n-$C_3H_7$ | |
| 2.084 | F | $CF_2Cl$ | i-$C_3H_7$ | |
| 2.085 | F | $CF_2Cl$ | sec-$C_4H_9$ | |
| 2.086 | F | $CF_2Cl$ | i-$C_4H_9$ | |
| 2.087 | F | $CHF_2$ | $CH_3$ | |
| 2.088 | F | $CHF_2$ | $C_2H_5$ | |
| 2.089 | F | $CHF_2$ | n-$C_3H_7$ | |
| 2.090 | F | $CHF_2$ | i-$C_3H_7$ | |
| 2.091 | F | $CHF_2$ | sec-$C_4H_9$ | |
| 2.092 | F | $CHF_2$ | i-$C_4H_9$ | |
| 2.093 | F | $CF_2CCl_3$ | $CH_3$ | |
| 2.094 | F | $CF_2CCl_3$ | $C_2H_5$ | |
| 2.095 | F | $CF_2CCl_3$ | n-$C_3H_7$ | |
| 2.096 | F | $CF_2CCl_3$ | i-$C_3H_7$ | |
| 2.097 | F | $CF_2CCl_3$ | sec-$C_4H_9$ | |
| 2.098 | F | $CF_2CCl_3$ | i-$C_4H_9$ | |
| 2.099 | F | $CCl_2CF_3$ | $CH_3$ | |
| 2.100 | F | $CCl_2CF_3$ | $C_2H_5$ | |
| 2.101 | F | $CCl_2CF_3$ | n-$C_3H_7$ | |
| 2.102 | F | $CCl_2CF_3$ | i-$C_3H_7$ | |
| 2.103 | F | $CCl_2CF_3$ | sec-$C_4H_9$ | |
| 2.104 | F | $CCl_2CF_3$ | i-$C_4H_9$ | |
| 2.105 | F | $C_2F_5$ | $CH_3$ | |
| 2.106 | F | $C_2F_5$ | $C_2H_5$ | Fp. 63-65°C |
| 2.107 | F | $C_2F_5$ | n-$C_3H_7$ | |
| 2.108 | F | $C_2F_5$ | i-$C_3H_7$ | |
| 2.109 | F | $C_2F_5$ | sec-$C_4H_9$ | |
| 2.110 | F | $C_2F_5$ | i-$C_4H_9$ | |
| 2.111 | F | $CF_2$-$CF_2Cl$ | $CH_3$ | |
| 2.112 | F | $CF_2$-$CF_2Cl$ | $C_2H_5$ | |
| 2.113 | F | $CF_2$-$CF_2Cl$ | n-$C_3H_7$ | |
| 2.114 | F | $CF_2$-$CF_2Cl$ | i-$C_3H_7$ | |
| 2.115 | F | $CF_2$-$CF_2Cl$ | sec-$C_4H_9$ | |
| 2.116 | F | $CF_2$-$CF_2Cl$ | i-$C_4H_9$ | |
| 2.117 | F | $CF_3$ | $C_2H_5$ | Fp. 89-91°C |
| 2.118 | F | $CF_3$ | i $C_3H_7$ | $n_D^{30}$ : 1,5313 |
| 2.119 | F | $CF_3$ | sec-$C_4H_9$ | |
| 2.120 | F | $CF_3$ | i-$C_4H_9$ | |

Tabelle 2 (Fortsetzung)

| Verb. No. | $R^1$ | $R^2$ | $R^3$ | phys. Daten |
|---|---|---|---|---|
| 2.121 | Cl | $CF_2Cl$ | $CH_3$ | |
| 2.122 | Cl | $CF_2Cl$ | $C_2H_5$ | |
| 2.123 | Cl | $CF_2Cl$ | $n-C_3H_7$ | |
| 2.124 | Cl | $CF_2Cl$ | $i-C_3H_7$ | |
| 2.125 | Cl | $CF_2Cl$ | $sec-C_4H_9$ | |
| 2.126 | Cl | $CF_2Cl$ | $i-C_4H_9$ | |
| 2.127 | Cl | $CHF_2$ | $CH_3$ | |
| 2.128 | Cl | $CHF_2$ | $C_2H_5$ | |
| 2.129 | Cl | $CHF_2$ | $n-C_3H_7$ | |
| 2.130 | Cl | $CHF_2$ | $i-C_3H_7$ | |
| 2.131 | Cl | $CHF_2$ | $sec-C_4H_9$ | |
| 2.132 | Cl | $CHF_2$ | $i-C_4H_9$ | |
| 2.133 | Cl | $CF_2CCl_3$ | $CH_3$ | |
| 2.134 | Cl | $CF_2CCl_3$ | $C_2H_5$ | |
| 2.135 | Cl | $CF_2CCl_3$ | $n-C_3H_7$ | |
| 2.136 | Cl | $CF_2CCl_3$ | $i-C_3H_7$ | |
| 2.137 | Cl | $CF_2CCl_3$ | $sec-C_4H_9$ | |
| 2.138 | Cl | $CF_2CCl_3$ | $i-C_4H_9$ | |
| 2.139 | Cl | $CCl_2CF_3$ | $CH_3$ | |
| 2.140 | Cl | $CCl_2CF_3$ | $C_2H_5$ | |
| 2.141 | Cl | $CCl_2CF_3$ | $n-C_3H_7$ | |
| 2.142 | Cl | $CCl_2CF_3$ | $i-C_3H_7$ | |
| 2.143 | Cl | $CCl_2CF_3$ | $sec-C_4H_9$ | |
| 2.144 | Cl | $CCl_2CF_3$ | $i-C_4H_9$ | |
| 2.145 | Cl | $C_2F_5$ | $CH_3$ | |
| 2.146 | Cl | $C_2F_5$ | $C_2H_5$ | |
| 2.147 | Cl | $C_2F_5$ | $n-C_3H_7$ | |
| 2.148 | Cl | $C_2F_5$ | $i-C_3H_7$ | |
| 2.149 | Cl | $C_2F_5$ | $sec-C_4H_9$ | |
| 2.150 | Cl | $C_2F_5$ | $i-C_4H_9$ | |
| 2.151 | Cl | $CF_2-CF_2Cl$ | $CH_3$ | |
| 2.152 | Cl | $CF_2-CF_2Cl$ | $C_2H_5$ | |
| 2.153 | Cl | $CF_2-CF_2Cl$ | $n-C_3H_7$ | |
| 2.154 | Cl | $CF_2-CF_2Cl$ | $i-C_3H_7$ | |
| 2.155 | Cl | $CF_2-CF_2Cl$ | $sec-C_4H_9$ | |
| 2.156 | Cl | $CF_2-CF_2Cl$ | $i-C_4H_9$ | |
| 2.157 | Cl | $CF_3$ | $C_2H_5$ | |
| 2.158 | Cl | $CF_3$ | $i\ C_3H_7$ | |
| 2.159 | Cl | $CF_3$ | $sec-C_4H_9$ | |
| 2.160 | Cl | $CF_3$ | $i-C_4H_9$ | |

Tabelle 2 (Fortsetzung)

| Verb. No. | $R^1$ | $R^2$ | $R^3$ | phys. Daten |
|---|---|---|---|---|
| 2.161 | Br | $CF_2Cl$ | $CH_3$ | |
| 2.162 | Br | $CF_2Cl$ | $C_2H_5$ | |
| 2.163 | Br | $CF_2Cl$ | $n-C_3H_7$ | |
| 2.164 | Br | $CF_2Cl$ | $i-C_3H_7$ | |
| 2.165 | Br | $CF_2Cl$ | $sec-C_4H_9$ | |
| 2.166 | Br | $CF_2Cl$ | $i-C_4H_9$ | |
| 2.167 | Br | $CHF_2$ | $CH_3$ | |
| 2.168 | Br | $CHF_2$ | $C_2H_5$ | |
| 2.169 | Br | $CHF_2$ | $n-C_3H_7$ | |
| 2.170 | Br | $CHF_2$ | $i-C_3H_7$ | |
| 2.171 | Br | $CHF_2$ | $sec-C_4H_9$ | |
| 2.172 | Br | $CHF_2$ | $i-C_4H_9$ | |
| 2.173 | Br | $CF_2CCl_3$ | $CH_3$ | |
| 2.174 | Br | $CF_2CCl_3$ | $C_2H_5$ | |
| 2.175 | Br | $CF_2CCl_3$ | $n-C_3H_7$ | |
| 2.176 | Br | $CF_2CCl_3$ | $i-C_3H_7$ | |
| 2.177 | Br | $CF_2CCl_3$ | $sec-C_4H_9$ | |
| 2.178 | Br | $CF_2CCl_3$ | $i-C_4H_9$ | |
| 2.179 | Br | $CCl_2CF_3$ | $CH_3$ | |
| 2.180 | Br | $CCl_2CF_3$ | $C_2H_5$ | |
| 2.181 | Br | $CCl_2CF_3$ | $n-C_3H_7$ | |
| 2.182 | Br | $CCl_2CF_3$ | $i-C_3H_7$ | |
| 2.183 | Br | $CCl_2CF_3$ | $sec-C_4H_9$ | |
| 2.184 | Br | $CCl_2CF_3$ | $i-C_4H_9$ | |
| 2.185 | Br | $C_2F_5$ | $CH_3$ | |
| 2.186 | Br | $C_2F_5$ | $C_2H_5$ | |
| 2.187 | Br | $C_2F_5$ | $n-C_3H_7$ | |
| 2.188 | Br | $C_2F_5$ | $i-C_3H_7$ | |
| 2.189 | Br | $C_2F_5$ | $sec-C_4H_9$ | |
| 2.190 | Br | $C_2F_5$ | $i-C_4H_9$ | |
| 2.191 | Br | $CF_2-CF_2Cl$ | $CH_3$ | |
| 2.192 | Br | $CF_2-CF_2Cl$ | $C_2H_5$ | |
| 2.193 | Br | $CF_2-CF_2Cl$ | $n-C_3H_7$ | |
| 2.194 | Br | $CF_2-CF_2Cl$ | $i-C_3H_7$ | |
| 2.195 | Br | $CF_2-CF_2Cl$ | $sec-C_4H_9$ | |
| 2.196 | Br | $CF_2-CF_2Cl$ | $i-C_4H_9$ | |
| 2.197 | Br | $CF_3$ | $C_2H_5$ | |
| 2.198 | Br | $CF_3$ | $i\ C_3H_7$ | |
| 2.199 | Br | $CF_3$ | $sec-C_4H_9$ | |
| 2.200 | Br | $CF_3$ | $i-C_4H_9$ | |
| 2.201 | H | $CF_3$ | $n-C_3H_7$ | Fp. 61-62°C |
| 2.202 | $CH_3$ | $CF_3$ | $n-C_3H_7$ | $n_D^{25}$: 1,5425 |
| 2.203 | F | $CF_3$ | $n-C_3H_7$ | |
| 2.204 | Cl | $CF_3$ | $n-C_3H_7$ | |
| 2.205 | Br | $CF_3$ | $n-C_3H_7$ | |

## Biologische Beispiele

### Beispiel B1: Pre-emergente Herbizid-Wirkung

Im Gewächshaus wird unmittelbar nach der Einsaat der Versuchspflanzen in Saatschalen die Erdoberfläche mit einer wässrigen Spritzbrühe entsprechend einer Aufwandmenge von 4 kg Wirksubstanz/Hektar behandelt. Die Saatschalen werden im Gewächshaus bei 22 - 25°C und 50 - 70 % relativer Luftfeuchtigkeit gehalten.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin.

In diesem Test zeigen die nachstehend genannten Verbindungen der Formel I gute bis sehr gute Herbizidwirkung bei den Unkräutern Setaria und Stellaria: Verbindungen Nos.: 1.001, 1.004, 1.007, 1.025, 1.026, 1.028, 1.037, 1.038, 1.039, 1.041, 1.044, 1.047, 1.066, 1.077, 1.078, 1.079, 1.117, 1.118, 1.201 und 1.202.

Beispiel B2: Selektive Herbizidwirkung für Wasserreis (verpflanzt)

Wasserunkräuter werden in Plastikgefässen (425 $cm^2$ Oberfläche, 5,0 1 Volumen) ausgesät. Zusätzlich wird Reis im Dreiblattstadium verpflanzt (verpflanzter Reis). Nach dem Verpflanzen wird bis zur Erdoberfläche mit Wasser aufgefüllt. 3 Tage nach der Saat wird der Wasserspiegel bis leicht über die Erdoberfläche erhöht (3-5 mm). Die Applikation der Prüfsubstanz erfolgt 3 Tage nach der Saat in einer Aufwandmenge von 1000 und 500 g/ha durch Injektion einer wässrigen Emulsion in das Wasser (das Applikationsvolumen entspricht dabei einer Aufwandmenge von 1400 l/ha). Die Pflanzgefässe werden dann im Gewächshaus unter optimalen Wachstumsbedingungen für die Einsaat aufgestellt, d.h. bei 25-30°C und hoher Luftfeuchtigkeit.

Die Versuch werden drei Wochen nach Applikation in einem neunstufigen Bonitierungsschema im Vergleich zur unbehandelten Kontrolle ausgewertet.

Boniturnoten von 1 bis 4 (insbesondere von 1 bis 3) weisen auf eine gute bis sehr gute Herbizidwirkung hin. Boniturnoten von 6 bis 9 (insbesondere von 7 bis 9) weisen auf eine gute Toleranz (insbesondere bei dem Reis) hin.

Dabei zeigen die nachstehend genannten Verbindungen der Formel I eine gute Herbizidwirkung gegen Echinochloa crus galli bei gleichzeitig guter Toleranz durch den Reis: Verb. No.: 1.001, 1.037 und 1.047.

Beispiel B3: Selektive Herbizidwirkung im Vorauflauf

Unmittelbar nach der Einsaat der Samen in Blumentöpfe von 12-15 cm Durchmesser wird die Oberfläche mit einer wässrigen Spritzbrühe, entsprechend einer Aufwandmenge von 1000 [g] AS/[ha], behandelt.

Die Töpfe werden im Gewächshaus bei einer Temperatur von 22-25°C und 50-70 % relativer Luftfeuchtigkeit belassen.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

In diesem Testmodell zeigen die nachstehend genannten Verbindungen der Formel I gute bis sehr gute Herbizidwirkung unter anderem gegen die nachstehend genannten Unkräuter:
Digitaria Sang., Echinochloa crus galli, Sorghum halep., Chenopodium Sp., Chrysanthenum leuc., Galium aparine, Viola tricolor und Veronica Sp.: 1.007, 1.025, 1.037, 1.038, 1.041, 1.044 und 1.106.

Weiterhin zeigen die Verbindungen 1.007, 1.025, 1.037 und 1.038 unter anderem gute bis sehr gute Herbizidwirkung gegen Lolium perenne, Alopecurus myos., Abutilon, Chenopodium Sp., Solanum nigrum und Stellaria.

Gute bis sehr gute Kulturpflanzenverträglichkeit findet sich bei den vorgenannten Verbindungen unter anderem bei Gerste, Mais, Soja, Baumwolle, Sonnenblume oder Raps.

Beispiel B4: Wuchshemmung bei Getreide

Die Pflanzen (z.B. Sommergerste der Sorte Iban) werden in 15 cm-Kunststofftöpfen mit steriler Landerde angesät und in der Klimakammer bei einer Tagestemperatur von 10-15°C und einer Nachttemperatur von 5-10° angezogen. Die Beleuchtungsdauer ist 13,5 Stunden pro Tag.

Ca. 34 Tage nach der Saat und dem Ausdünnen auf 4 Pflanzen/Topf erfolgt die Applikation mit 0,3 bis 3 kg Wirkstoff/ha, in der Regel 25%-ig formuliert und in wässriger Spritzbrühe. Die Wasseraufwandmenge ist ca. 500 l/ha. Nach der Applikation werden die Pflanzen im Gewächshaus bei einer Tagestemperatur von mindestens 10°C aufgestellt. Die Beleuchtungsdauer ist mind. 13,5 Stunden/Tag.

Ca. 28 Tage nach der Behandlung findet die Auswertung statt. Hierbei wird die Höhe des Neuzuwachses dargestellt.

Die geprüften Verbindungen der Formel I bewirken eine Reduzierung des Neuzuwachses im Vergleich zur unbehandelten Kontrolle.

Beispiel B5; Wuchshemmung bei Gräsern mit Klee

Eine Mischung von Gräsern /z.B. Poa, Festuca, Lolium, Bromus, Cynosurus) und Klee (Trifolium pratense/repens) wird in 15 cm-Kunststofftöpfen mit steriler Landerde angesät und im Gewächshaus bei einer Tagestemperatur von 21°C und einer Nachttemperatur von 17°C angezogen. Die Beleuchtungsdauer ist 13,5 Stunden/Tag bei einer Lichtintensität von mind. 7000 Lux. Nach dem Auflauf werden die Pflanzen wöchentlich auf ca. 6 cm Höhe zurückgeschnitten. Ca. 42 Tage nach der Saat und 1 Tag nach dem letzten Schnitt erfolgt die Applikation mit 0,3 bis 3 kg Wirkstoff/ha, in der Regel 25%-ig formuliert und in wässriger Spritzbrühe. Die Wasseraufwandmenge ist ca. 500 l/ha.

Ca. 3 Wochen nach der Behandlung findet die Auswertung statt. Hierbei wird die Höhe des Neuzuwachses gemessen.

Die geprüften Verbindungen der Formel I bewirken eine Reduktion des Neuzuwachses im Vergleich zur unbehandelten Kontrolle.

Beispiel B6: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabelle 1 besprüht. 21 Tage nach Applikation wird das Wachstum des Getreides beurteilt. Die behandelten Pflanzen weisen im Vergleich zu unbehandelten Kontrollen eine Verringerung des Neuzuwachses, sowie teilweise eine Zunahme der Stengeldurchmesser auf.

Beispiel B7: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabelle 1 besprüht. Die Wirkstoffmenge beträgt umgerechnet bis zu 500 g Aktivsubstanz pro Hektar. 21 Tage nach Applikation wird das Wachstum der Gräser beurteilt.

Die geprüften Verbindungen der Tabelle 1 bewirken eine Reduzierung des Neuzuwachses im Vergleich zur unbehandelten Kontrolle.

Formulierungsbeispiele

Beispiel F1: Formulierungsbeispiele für Wirkstoffe der Formel I' (% = Gewichtsprozent)

| a) Emulsionskonzentrate | a) | b) | c) |
|---|---|---|---|
| Wirkstoff aus Tabelle 1 | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenol-polyäthylenglykoläther (30 mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) |
| --- | --- | --- | --- |
| Wirkstoff aus Tabelle 1 | 80 % | 10 % | 5 % |
| Aethylenglykol-monomethyläther | 20 % | - | - |
| Polyäthylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | a) | b) |
| --- | --- | --- |
| Wirkstoff aus Tabelle 1 | 5 % | 10 % |
| Kaolin | 94 % | - |
| Hochdisperse Kieselsäure | 1 % | |
| Attapulgit | - | 90 % |

Eine Wirkstofflösung wird auf den Träger aufgesprüht und das Lösungsmittel anschliessend im Vakuum abgedampft.

| d) Stäubemittel | a) | b) |
| --- | --- | --- |
| Wirkstoff aus Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoffe erhält man gebrauchsfertiges Stäubemittel.

| e) Spritzpulver | a) | b) |
| --- | --- | --- |
| Wirkstoff aus Tabelle 1 | 20 % | 60 % |
| Na-Ligninsulfonat | 5 % | 5 % |
| Na-Laurylsulfat | - | 6 % |
| Octylphenolpolyäthylenglykoläther (7-8 Mol Ae) | - | 2 % |
| Hochdisperse Kieselsäure | 5 % | 27 % |
| Kaolin | 70 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

f) Extruder Granulat

| | |
|---|---|
| Wirkstoff aus Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

g) Umhüllungs-Granulat

| | |
|---|---|
| Wirkstoff aus Tabelle 1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

h) Suspensions-Konzentrat

| | |
|---|---|
| Wirkstoff aus Tabelle 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | ad 100 % |

Der Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Patentansprüche**

**Patentansprüche für folgende Vertragsstaaten : AT, BE, CH, DE, FR, GB, GR, IT, LI, LU, NL, SE**

**1.** Harnstoffe der Formel I

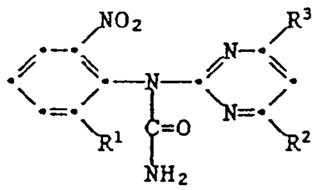

(I),

worin

$R^1$ Wasserstoff, Methyl, Fluor, Chlor oder Brom,

$R^2$ $C_1$-$C_2$-Halogenalkyl mit mindestens 2 Fluoratomen und

$R^3$ $C_1$-$C_4$-Alkyl bedeutet, mit der Massgabe, dass $R^2$ nicht Trifluormethyl bedeutet, wenn $R^3$ für Methyl steht, sowie die Salze und Additionsverbindungen der Harnstoffe der Formel I mit Säuren, Basen oder Komplexbildnern.

**2.** Harnstoffe der Formel I gemäss Anspruch 1,worin

$R^1$ Wasserstoff, Methyl, Fluor, Chlor oder Brom,

$R^2$ Chlordifluormethyl, Difluormethyl, 1,1-Difluor-2,2,2-trichlorethyl, 1,1-Dichlor-2,2,2-trifluorethyl, Pentafluorethyl, 1,1,2,2-Tetrafluor-2-chlorethyl oder Trifluormethyl und

$R^3$ $C_1$-$C_4$-Alkyl bedeutet.

**3.** Harnstoffe der Formel I gemäss Anspruch 1 oder 2, worin

$R^1$ Wasserstoff, Methyl, Fluor, Chlor oder Brom,

$R^2$ Chlordifluormethyl, Difluormethyl, 1,1-Difluor-2,2,2-trichlorethyl, 1,1-Dichlor-2,2,2-trifluorethyl, Pentafluorethyl, 1,1,2,2-Tetrafluor-2-chlorethyl oder Trifluormethyl und

$R^3$ Methyl, Ethyl, n-Propyl, iso-Propyl, sec-Butyl oder iso-Butyl bedeutet.

**4.** 2-[N-Carbamyl-N-(6-methyl-2-nitrophenyl)-amino]-4-trifluormethyl-6-ethyl-pyrimidin oder 2-[N-Carbamyl-N-(2-nitrophenyl)-amino]-4-trifluormethyl-6-ethyl-pyrimidin als Verbindungen der Formel I gemäss Anspruch 2.

**5.** Verfahren zur Herstellung von Harnstoffen der Formel I gemäss einem der Ansprüche 1 bis 4, dadurch gekennzeichnet, dass man

a) ein Anilin der Formel II mit Phosgen zu einem Carbaminchlorid der Formel III umsetzt und dieses in einer zweiten Stufe mit $NH_3$ zu

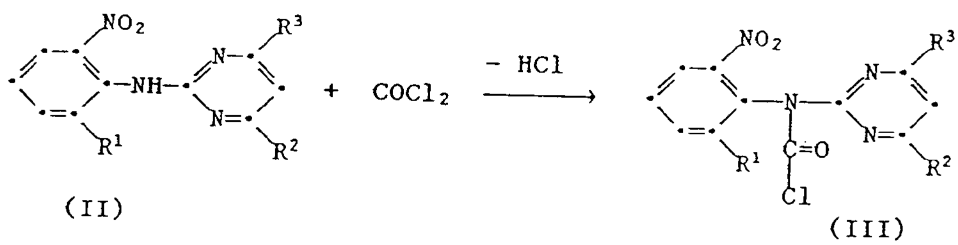


(II) (III)

$$III + NH_3 \xrightarrow{-\ HCl} I$$

einem Harnstoff der Formel I umsetzt oder

b) ein Anilin der Formel II mit Halogensulfonylisocyanat zu einem Halogensulfonylharnstoff der Formel IV umsetzt

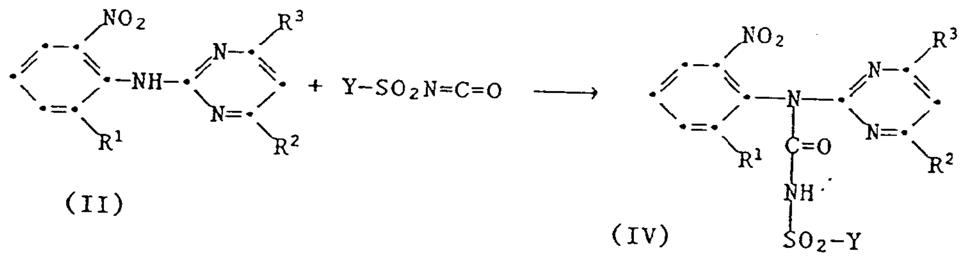


(II) (IV)

$$IV + 2H_2O \xrightarrow[- SO_4H_2]{- HY} I$$

und diesen in einer zweiten Stufe oder direkt zu einer Verbindung der Formel I hydrolisiert, wobei Y für eine unter den Reaktionsbedingungen abspaltbare Gruppe wie Halogen, vorzugsweise Chlor, steht oder

c) einen Sulfonylharnstoff der Formel V unter Enwirkung einer wässrigen Base zu einem Harnstoff der Formel I umlagert

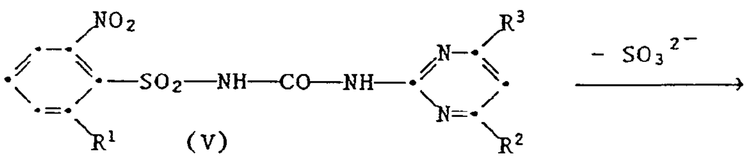


(V) (I),

wobei als wässrige Basen vorzugsweise NaOH/Wasser oder KOH/Wasser verwendet werden.

**6.** Harnstoffe der Formel IV

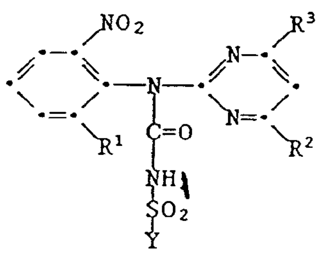

(IV),

worin

| | |
|---|---|
| Y | Halogen, |
| $R^1$ | Wasserstoff, Methyl, Fluor, Chlor oder Brom, |
| $R^2$ | $C_1$-$C_2$-Halogenalkyl mit mindestens 2 Fluoratomen und |
| $R^3$ | $C_1$-$C_4$-Alkyl |

bedeutet,

mit der Massgabe, dass $R^2$ nicht Trifluormethyl bedeutet, wenn $R^3$ für Methyl steht.

**7.** Verfahren zur Herstellung von Harnstoffen der Formel IV gemäss Anspruch 6, dadurch gekennzeichnet, dass man ein Anilin der Formel II mit Halogensulfonylisocyanat umsetzt

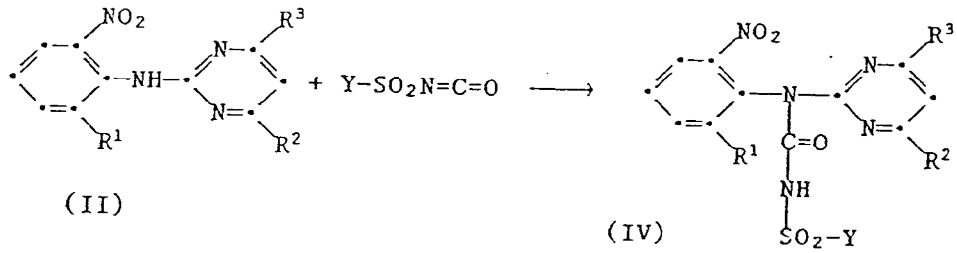
(II)
(IV)

8. Herbizides Mittel, enthaltend als Wirksubstanz eine Verbindung der Formel I, gemäss einem der Ansprüche 1 bis 4, neben weiteren Hilfs-und/oder Trägerstoffen.

9. Pflanzenwuchsregularorisches Mittel, enthaltend als Wirksubstanz eine Verbindung der Formel I, gemäss einem der Ansprüche 1 bis 4, neben weiteren Hilfs- und/oder Trägerstoffen.

10. Verfahren zur Bekämpfung unerwünschten Pflanzenwuchses, dadurch gekennzeichnet, dass man eine herbizid wirksame Menge einer Verbindung, gemäss einem der Ansprüche 1 bis 4, oder eines Mittels gemäss Anspruch 8 auf die zu bekämpfende Pflanze oder deren Lebensraum einwirken lässt.

11. Verfahren zur Beeinflussung des Pflanzenwuchses, dadurch gekennzeichnet, dass man eine pflanzenwuchsregulatorisch wirksame Menge einer Verbindung, gemäss einem der Ansprüche 1 bis 4 oder eines Mittels, gemäss Anspruch 9 auf die Pflanze oder deren Lebensraum einwirken lässt.

**Patentansprüche für folgenden Vertragsstaat : ES**

1. Verfahren zur Herstellung von Harnstoffen der Formel I

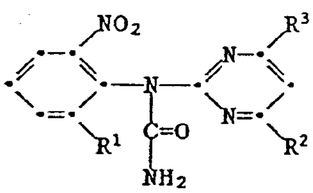
(I),

worin
$R^1$ Wasserstoff, Methyl, Fluor, Chlor oder Brom,
$R^2$ $C_1$-$C_2$-Halogenalkyl mit mindestens 2 Fluoratomen und
$R^3$ $C_1$-$C_4$-Alkyl
bedeutet,
mit der Massgabe, dass $R^2$ nicht Trifluormethyl bedeutet, wenn $R^3$ für Methyl steht, sowie die Salze und Additionsverbindungen der Harnstoffe der Formel I mit Säuren, Basen oder Komplexbildnern, dadurch gekennzeichnet, dass man

a) ein Anilin der Formel II mit Phosgen zu einem Carbaminchlorid der Formel III umsetzt und dieses in einer zweiten Stufe mit $NH_3$ zu

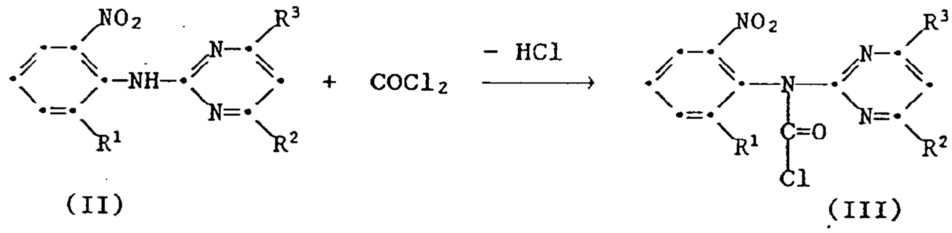


$$III + NH_3 \xrightarrow{-\ HCl} I$$

einem Harnstoff der Formel I umsetzt oder

b) ein Anilin der Formel II mit Halogensulfonylisocyanat zu einem Halogensulfonylharnstoff der Formel IV umsetzt

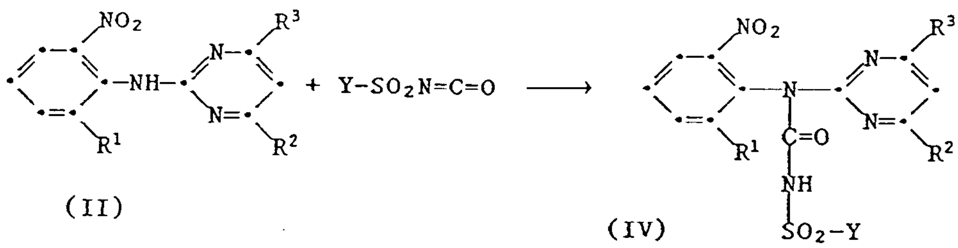


$$IV + 2H_2O \xrightarrow[-\ SO_4H_2]{-\ HY} I$$

und diesen in einer zweiten Stufe oder direkt zu einer Verbindung der Formel I hydrolisiert, wobei Y für eine unter den Reaktionsbedingungen abspaltbare Gruppe wie Halogen, vorzugsweise Chlor, steht oder

c) einen Sulfonylharnstoff der Formel V unter Einwirkung einer wässrigen Base zu einem Harnstoff der Formel I umlagert

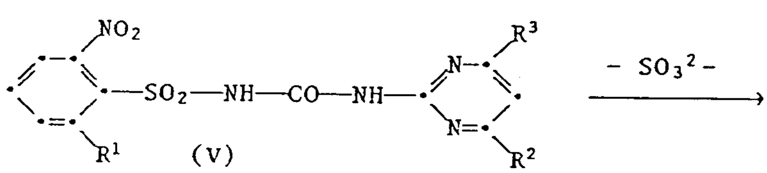


(I),

wobei als wässrige Basen vorzugsweise NaOH/Wasser oder KOH/Wasser verwendet werden und gegebenenfalls die nach den vorgenannten Verfahren erhaltene Verbindung der Formel I mit einer Säure, einer Base oder einem Komplexbildner in ein Salz oder ein Additionsprodukt überführt.

**2.** Verfahren gemäss Anspruch 1 zur Herstellung von 2-[N-Carbamyl-N-(6-methyl-2-nitrophenyl)-amino]-4-trifluormethyl-6-ethyl-pyrimidin oder 2-[N-Carbamyl-N-(2-nitrophenyl)-amino]-4-trifluormethyl-6-ethyl-pyrimidin.

**3.** Verfahren zur Herstellung von Harnstoffen der Formel IV

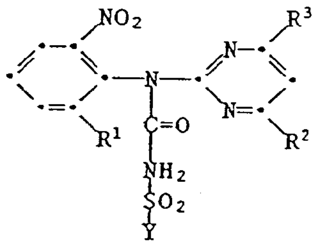

(IV),

worin

Y Halogen,

$R^1$ Wasserstoff, Methyl, Fluor, Chlor oder Brom,

$R^2$ $C_1$-$C_2$-Halogenalkyl mit mindestens 2 Fluoratomen und

$R^3$ $C_1$-$C_4$-Alkyl

bedeutet,

mit der Massgabe, dass $R^2$ nicht Trifluormethyl bedeutet, wenn $R^3$ für Methyl steht, dadurch gekennzeichnet, dass man ein Anilin der Formel II mit Halogensulfonylisocyanat umsetzt

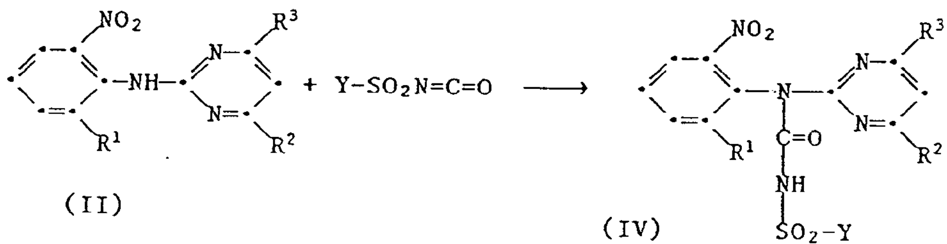

(II) (IV)

4. Verfahren zur Bekämpfung unerwünschten Pflanzenwuchses, dadurch gekennzeichnet, dass man eine herbizid wirksame Menge einer Verbindung der Formel I

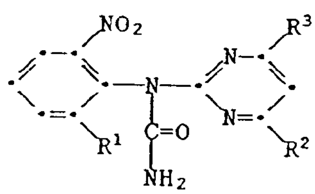

(I),

worin

$R^1$ Wasserstoff, Methyl, Fluor, Chlor oder Brom,

$R^2$ $C_1$-$C_2$-Halogenalkyl mit mindestens 2 Fluoratomen und

$R^3$ $C_1$-$C_4$-Alkyl

bedeutet,

mit der Massgabe, dass $R^2$ nicht Trifluormethyl bedeutet, wenn $R^3$ für Methyl steht, sowie die Salze und Additionsverbindungen der Harnstoffe der Formel I mit Säuren, Basen oder Komplexbildnern auf die zu bekämpfende Pflanze oder deren Lebensraum einwirken lässt.

5. Verfahren zur Beeinflussung des Pflanzenwuchses, dadurch gekennzeichnet, dass man eine pflanzenwuchsregulatorisch wirksame Menge einer Verbindung der Formel I

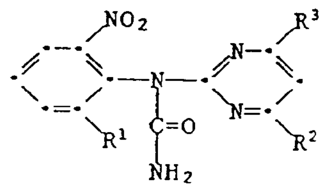

(I),

worin
$R^1$ Wasserstoff, Methyl, Fluor, Chlor oder Brom,
$R^2$ $C_1$-$C_2$-Halogenalkyl mit mindestens 2 Fluoratomen und
$R^3$ $C_1$-$C_4$-Alkyl
bedeutet,
mit der Massgabe, dass $R^2$ nicht Trifluormethyl bedeutet, wenn $R^3$ für Methyl steht, sowie die Salze und Additionsverbindungen der Harnstoffe der Formel I mit Säuren, Basen oder Komplexbildnern auf die Fflanze oder deren Lebensraum einwirken lässt.

6. Verfalren zur Herstellung eines herbizid oder pflanzenwuchsregulatorisch wirksamen Mittels, dadurch gekennzeichnet, dass man eine Verbindung der Formel I

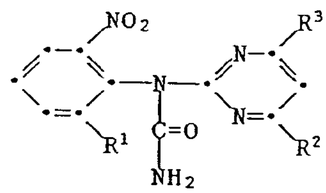

(I),

worin
$R^1$ Wasserstoff, Methyl, Fluor, Chlor oder Brom,
$R^2$ $C_1$-$C_2$-Halogenalkyl mit mindestens 2 Fluoratomen und
$R^3$ $C_1$-$C_4$-Alkyl
bedeutet,
mit der Massgabe, dass $R^2$ nicht Trifluormethyl bedeutet, wenn $R^3$ für Methyl steht, sowie die Salze und Additionsverbindungen der Harnstoffe der Formel I mit Säuren, Basen oder Komplexbildnern mit in der Formulierungstechnik gebräuchlichen Hilfs- und/oder Trägerstoffen vermischt.

7. Verfahren zur Bekämpfung unerwünschten Pflanzenwuchses oder zur Beeinflussung des Pflanzenwuchses, dadurch gekennzeichnet, dass man eine herbizid wirksame oder eine pflanzenwuchsregulatorisch wirksame Menge eines Mittels gemäss Anspruch 6 auf die zu bekämpfende Pflanze oder deren Lebensraum einwirken lässt.

8. Herbizides Mittel, enthaltend eine Verbindung der Formel I

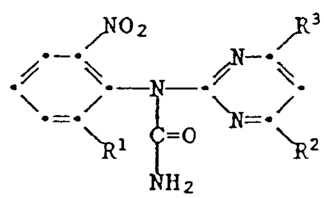

(I),

worin
$R^1$ Wasserstoff, Methyl, Fluor, Chlor oder Brom,
$R^2$ $C_1$-$C_2$-Halogenalkyl mit mindestens 2 Fluoratomen und
$R^3$ $C_1$-$C_4$-Alkyl
bedeutet,
mit der Massgabe, dass $R^2$ nicht Trifluormethyl bedeutet, wenn $R^3$ für Methyl steht, dadurch gekennzeichnet, dass es 0,1 bis 80% Wirkstoff der Formel I, 1 bis 99,9% eines festen oder flüssigen Zusatzstoffes und 0 bis 25% eines Tensides enthält.

## Claims

**Claims for the following Contracting States : AT, BE, CH, DE, FR, GB, GR, IT, LI, LU, NL, SE**

**1.** A urea of formula I

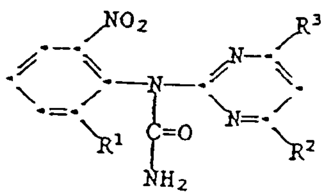

(I)

in which

$R^1$ is hydrogen, methyl, fluorine, chlorine or bromine,
$R^2$ is $C_1$-$C_2$haloalkyl having at least 2 fluorine atoms, and
$R^3$ is $C_1$-$C_4$alkyl,

with the proviso that $R^2$ is not trifluoromethyl when $R^3$ is methyl, or a salt or an addition compound of a urea of formula I with an acid, a base or a complex former.

**2.** A urea of formula I according to claim 1, in which $R^1$ is hydrogen, methyl, fluorine, chlorine or bromine, $R^2$ is chlorodifluoromethyl, difluoromethyl, 1,1-difluoro-2,2,2-trichloroethyl, 1,1-dichloro-2,2,2-trifluoroethyl, pentafluoroethyl, 1,1,2,2-tetrafluoro-2-chloroethyl or trifluoromethyl and
$R^3$ is $C_1$-$C_4$alkyl.

**3.** A urea of formula I according to claim 1 or 2, in which
$R^1$ is hydrogen, methyl, fluorine, chlorine or bromine,
$R^2$ is chlorodifluoromethyl, difluoromethyl, 1,1-difluoro-2,2,2-trichloroethyl, 1,1-dichloro-2,2,2-trifluoroethyl, pentafluoroethyl, 1,1,2,2-tetrafluoro-2-chloroethyl or trifluoromethyl and
$R^3$ is methyl, ethyl, n-propyl, isopropyl, sec-butyl or isobutyl.

**4.** 2-[N-carbamoyl-N-(6-methyl-2-nitrophenyl)-amino]-4-trifluoromethyl -6-ethyl-pyrimidine or 2-[N-carbamoyl-N-(2-nitrophenyl)-amino]-4-trifluoromethyl-6-ethyl-pyrimidine as a compound of formula I according to claim 2.

**5.** A process for the preparation of a urea of formula I according to any one of claims 1 to 4, which comprises
a) reacting an aniline of formula II with phosgene to form a carbamoyl chloride of formula III and reacting this with $NH_3$ in a second step

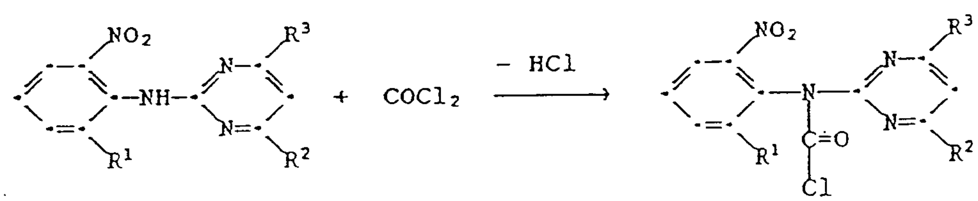


(II) (III)

$$III + NH_3 \xrightarrow{-\ HCl} I$$

to form a urea of formula I, or

b) reacting an aniline of formula II with a halosulfonyl isocyanate to form a halosulfonylurea of formula IV

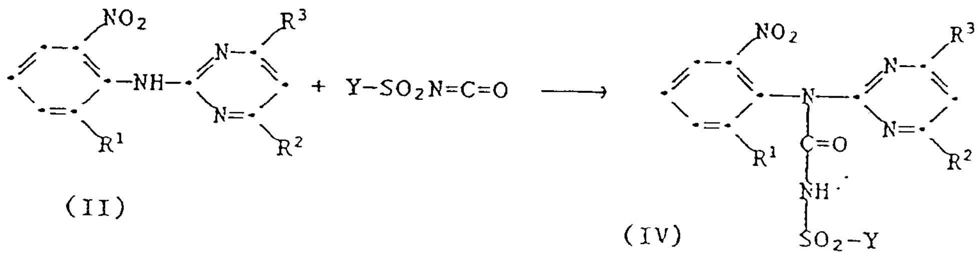

(II) (IV)

$$IV + 2H_2O \xrightarrow[- SO_4H_2]{- HY} I$$

and hydrolysing this in a second step, or directly, to a compound of formula I, Y being a group that can be removed under the reaction conditions, such as halogen, preferably chlorine, or

c) rearranging a sulfonylurea of formula V, under the action of an aqueous base, to form a urea of formula I

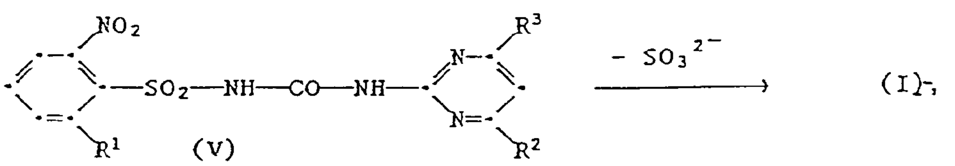


(V) (I),

NaOH/water or KOH/water preferably being used as aqueous base.

6. A urea of formula IV

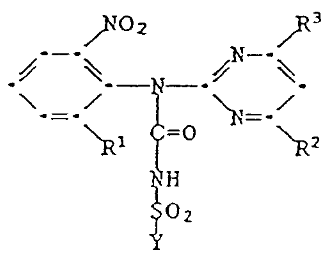

(IV)

in which

Y is halogen,

$R^1$ is hydrogen, methyl, fluorine, chlorine or bromine,

$R^2$ is $C_1$-$C_2$haloalkyl having at least 2 fluorine atoms, and

$R^3$ is $C_1$ -$C_4$alkyl,

with the proviso that $R^2$ is not trifluoromethyl when $R^3$ is methyl.

7. A process for the preparation of a urea of formula IV according to claim 6, which comprises reacting an aniline of formula II with a halosulfonyl isocyanate

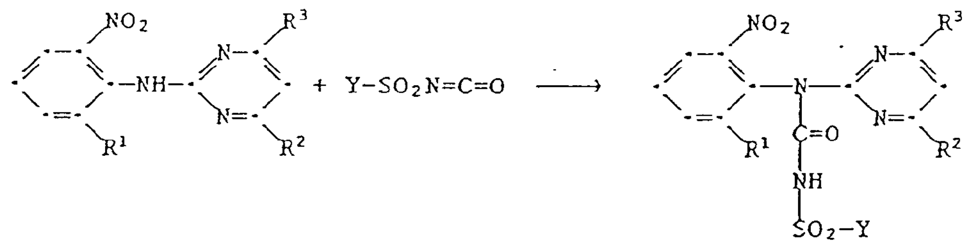

(II) (IV).

8. A herbicidal composition comprising as active ingredient a compound of formula I according to any one of claims 1 to 4 together with further adjuvants and/or carriers.

9. A plant growth-regulating composition comprising as active ingredient a compound of formula I according to any one of claims 1 to 4 together with further adjuvants and/or carriers.

10. A method of controlling undesired plant growth which comprises allowing a herbicidally effective amount of a compound according to any one of claims 1 to 4, or of a composition according to claim 8, to act on the plant to be controlled or the locus thereof.

11. A method of influencing plant growth which comprises allowing to act on the plant or the locus thereof an amount of a compound according to any one of claims 1 to 4, or of a composition according to claim 9, that is effective in regulating plant growth.

**Claims for the following Contracting State : ES**

1. A process for the preparation of a urea of formula I

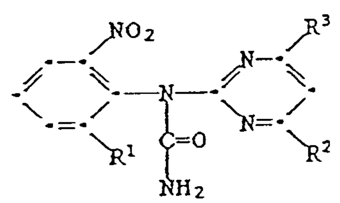

(I)

in which

$R^1$ is hydrogen, methyl, fluorine, chlorine or bromine,
$R^2$ is $C_1$-$C_2$haloalkyl having at least 2 fluorine atoms, and
$R^3$ is $C_1$-$C_4$alkyl,

with the proviso that $R^2$ is not trifluoromethyl when $R^3$ is methyl, or a salt or an addition compound of a urea of formula I with an acid, a base or a complex former, which comprises

a) reacting an aniline of formula II with phosgene to form a carbamoyl chloride of formula III and reacting this with $NH_3$ in a second step

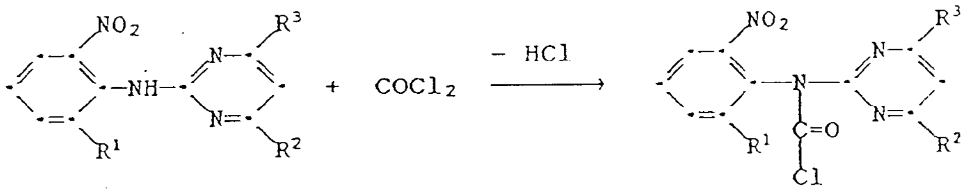


(II) (III)

$$III + NH_3 \xrightarrow{-\ HCl} I$$

to form a urea of formula I, or

b) reacting an aniline of formula II with a halosulfonyl isocyanate to form a halosulfonylurea of formula IV

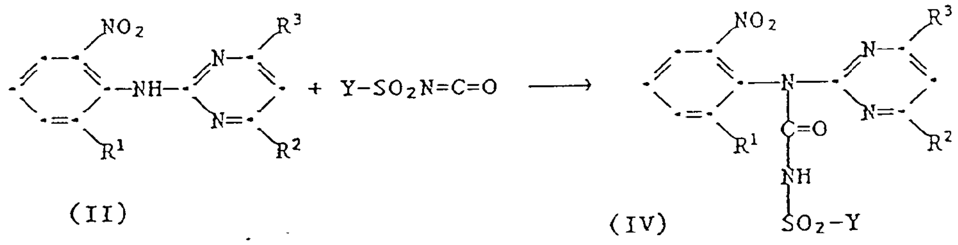


(II) (IV)

$$IV + 2H_2O \xrightarrow[-\ SO_4H_2]{-\ HY} I$$

and hydrolysing this in a second step, or directly, to a compound of formula I, Y being a group that can be removed under the reaction conditions, such as halogen, preferably chlorine, or

c) rearranging a sulfonylurea of formula V, under the action of an aqueous base, to form a urea of formula I

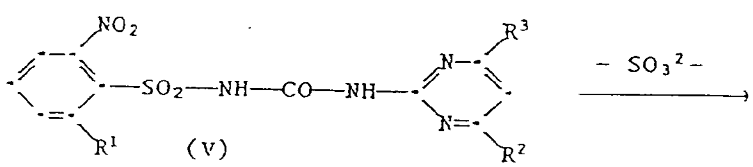


(V) (I), ,

NaOH/water or KOH/water preferably being used as aqueous base, and optionally converting the compound of formula I obtainable according to the above processes into a salt or an addition product with an acid, a base or a complex former.

2. A process according to claim 1 for the preparation of 2-[N-carbamoyl-N-(6-methyl-2-nitrophenyl)-amino]-4-tri-fluoromethyl-6-ethyl-pyrimidine or 2-[N-carbamoyl-N-(2-nitrophenyl)amino]-4-trifluoromethyl-6-ethyl-pyrimidine.

3. A process for the preparation of a urea of formula IV

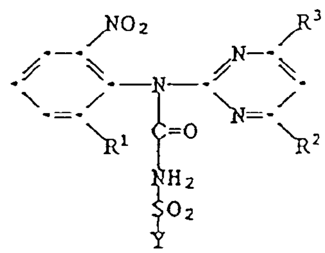

(IV)

in which

Y is halogen,

$R^1$ is hydrogen, methyl, fluorine, chlorine or bromine,

$R^2$ is $C_1$-$C_2$haloalkyl having at least 2 fluorine atoms, and

$R^3$ is $C_1$-$C_4$alkyl,

with the proviso that $R^2$ is not trifluoromethyl when $R^3$ is methyl, which comprises reacting an aniline of formula II with a halosulfonyl isocyanate

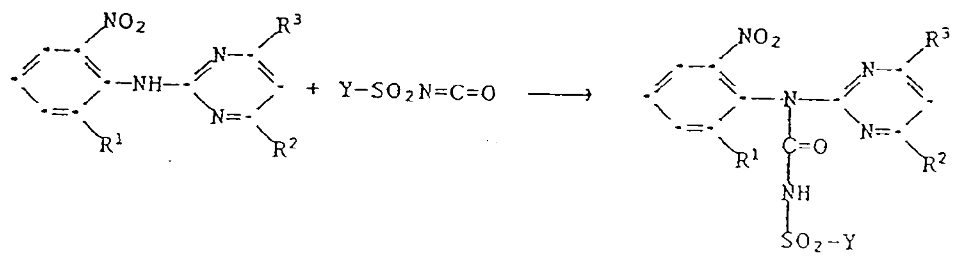

(II) (IV).

4. A method of controlling undesired plant growth, which comprises allowing to act on the plant to be controlled or the locus thereof a herbicidally effective amount of a compound of formula I

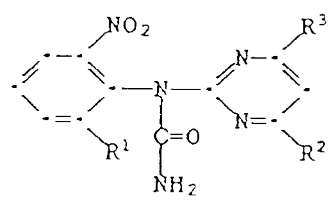

(I)

in which

$R^1$ is hydrogen, methyl, fluorine, chlorine or bromine,

$R^2$ is $C_1$-$C_2$haloalkyl having at least 2 fluorine atoms, and

$R^3$ is $C_1$ -$C_4$alkyl,

with the proviso that $R^2$ is not trifluoromethyl when $R^3$ is methyl, or of a salt or of an addition compound of a urea of formula I with an acid, a base or a complex former.

5. A method of influencing plant growth, which comprises allowing to act on the plant or the locus thereof an amount that is effective in regulating plant growth of a compound of formula I

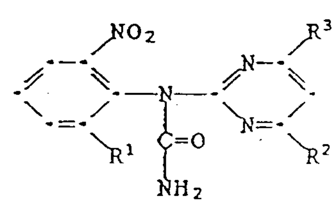

(I)

in which
$R^1$ is hydrogen, methyl, fluorine, chlorine or bromine,
$R^2$ is $C_1$ -$C_2$haloalkyl having at least 2 fluorine atoms, and
$R^3$ is $C_1$-$C_4$alkyl,
with the proviso that $R^2$ is not trifluoromethyl when $R^3$ is methyl, or of a salt or of an addition compound of a urea of formula I with an acid, a base or a complex former.

6. A process for the preparation of a composition that is herbicidally effective or effective in regulating plant growth which comprises mixing a compound of formula I

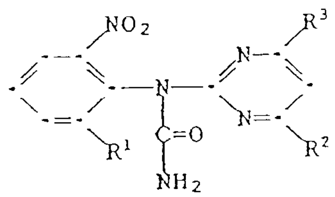

(I)

in which
$R^1$ is hydrogen, methyl, fluorine, chlorine or bromine,
$R^2$ is $C_1$-$C_2$haloalkyl having at least 2 fluorine atoms, and
$R^3$ is $C_1$-$C_4$alkyl,
with the proviso that $R^2$ is not trifluoromethyl when $R^3$ is methyl, or a salt or an addition compound of a urea of formula I with an acid, a base or a complex former, with adjuvants and/or carriers that are customary in formulation technology.

7. A method of controlling undesired plant growth or of influencing plant growth which comprises allowing to act on the plant to be controlled or the locus thereof a herbicidally effective amount, or an amount that is effective in regulating plant growth, of a composition according to claim 6.

8. A herbicidal composition comprising a compound of formula I

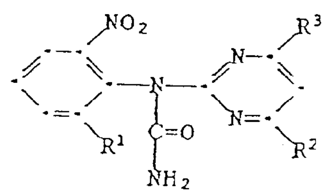

(I)

in which
$R^1$ is hydrogen, methyl, fluorine, chlorine or bromine,
$R^2$ is $C_1$-$C_2$haloalkyl having at least 2 fluorine atoms, and
$R^3$ is $C_1$-$C_4$alkyl,
with the proviso that $R^2$ is not trifluoromethyl when $R^3$ is methyl, which composition comprises from 0.1 to 80 % of active ingredient of formula I, from 1 to 99.9 % of a solid or liquid adjuvant and from 0 to 25 % of a surfactant.

## Revendications

**Revendications pour les Etats contractants suivants : AT, BE, CH, DE, FR, GB, GR, IT, LI, LU, NL, SE**

1. Urées de formule I

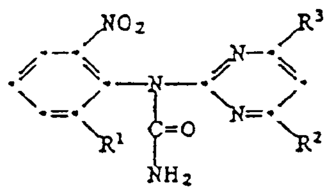

(I),

dans laquelle

$R^1$ représente un hydrogène, méthyle, fluor, chlore ou brome,

$R^2$ représente un halogènalcoyle en $C_{1-2}$ avec au moins 2 atomes de fluor et $R^4$ représente un alcoyle en $C_{1-4}$,

avec cette précision que $R^2$ ne représente pas un trifluorométhyle lorsque $R^3$ représente un méthyle, ainsi que les sels et composés d'addition désirés de formule I avec des acides, des bases ou des formateurs de complexes.

**2.** Urées de formule I selon la revendication 1, où

$R^1$ représente un hydrogène, méthyle, fluor, chlore ou brome,

$R^2$ représente un chlorodifluorométhyle, diifluorométhyle, 1,1-difluoro-2,2,2-trichloréthyle, 1,1-dichloro-2,2,2-trifluoroéthyle, pentafluoroéthyle, 1,1,2,2-tétrafluoro-2-chloroéthyle ou un trifluorométhyle, et

$R^3$ représente un alcoyle en $C_{1-4}$.

**3.** Urées de formule I selon les revendications 1 ou 2, où

$R^1$ représente un hydrogène, méthyle, fluor, chlore ou brome,

$R^2$ représente un chlorodifluorométhyle, difluorométhyle, 1,1-difluoro-2,2,2-trichloroéthyle, 1,1-dichloro-2,2,2-trifluoroéthyle, pentafluoroéthyle, 1,1,2,2-tétrafluoro-2-chloroéthyle ou trifluorométhyle et

$R^3$ représente un méthyle, éthyle, n-propyle, isopropyle, sec.-butyle ou iso-butyle.

**4.** 2-[N-carbamyl-N-(6-méthyl-2-nitrophényl)amino]-4-trifluorométhyl-6-éthyl-pyrimidine ou 2-[N-carbamyl -N-(2-nitrophényl)-amino]-4-trifluorométhyl-6-éthyl-pyrimidine comme composés de formule I selon la revendication 2.

**5.** Procédé de préparation d'urées de formule I selon l'une des revendications 1 à 4, caractérisé en ce que l'on procède :

a) en faisant réagir une aniline de formule II avec du phosgène pour donner un chlorure de carbamine de formule III et en faisant réagir celui-ci dans une seconde étape avec $NH_3$ pour donner

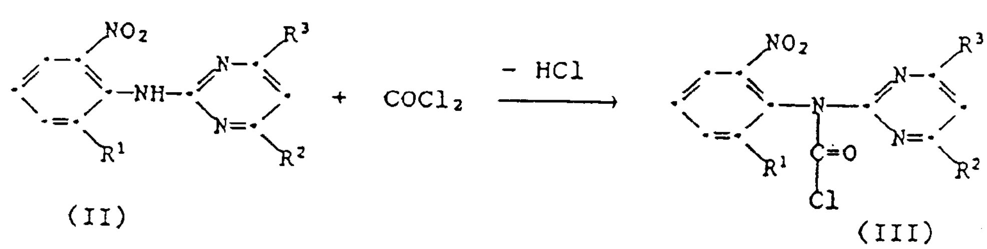


(II) (III)

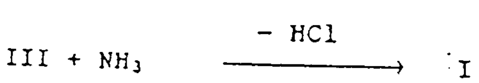


ou urée de formule I ou

b) en faisant réagir une aniline de formule II avec un isocyanate d'halogènesulfonyle pour donner une halogènesulfonylurée de formule IV

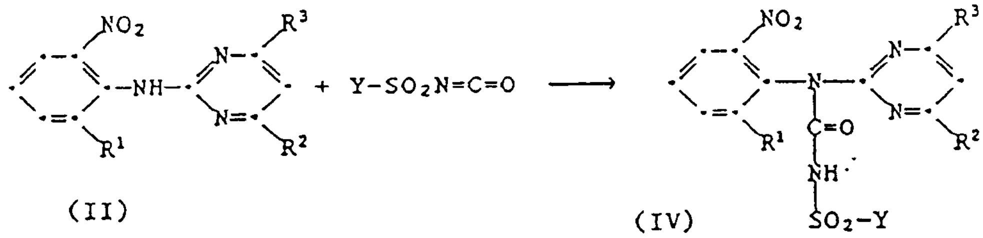


(II) (IV)

$$IV + 2H_2O \xrightarrow[- SO_4H_2]{- HY} I$$

et dans une seconde étape en hydrolysant directement celle-ci pour donner un composé de formule I, où Y représente un groupe séparable dans les conditions de la réaction, comme halogène, de préférence chlore, ou

c) en transposant une sulfonylurée de formule V sous l'action d'une base aqueuse pour donner une urée de formule I

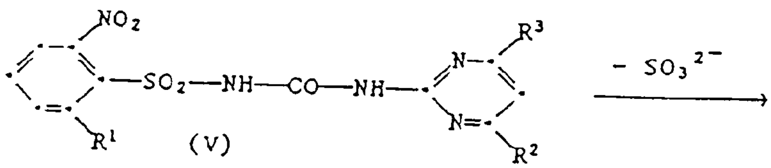


(V) (I),

où on utilise comme bases aqueuses de préférence NaOH/eau ou KOH/eau.

6. Urées de formule IV

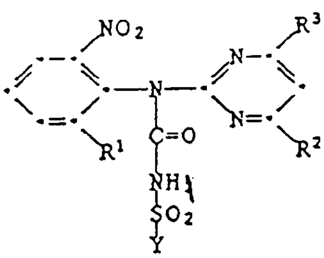

(IV),

ou

| | |
|---|---|
| Y | représente un halogène, |
| $R^1$ | représente un hydrogène, méthyle, fluor, chlore ou brome, |
| $R^2$ | représente un halogènalcoyle en $C_{1-2}$ avec deux atomes de fluor au moins, et |
| $R^3$ | représente un alcoyle en $C_{1-4}$, |

avec cette précision que $R^2$ ne représente pas un trifluorométhyle, lorsque $R^3$ représente un méthyle.

7. Procédé de préparation d'urées de formule IV selon la revendication 6, caractérisé en ce qu'on fait réagir une aniline de formule II avec un isocyanate d'halogènesulfonyle

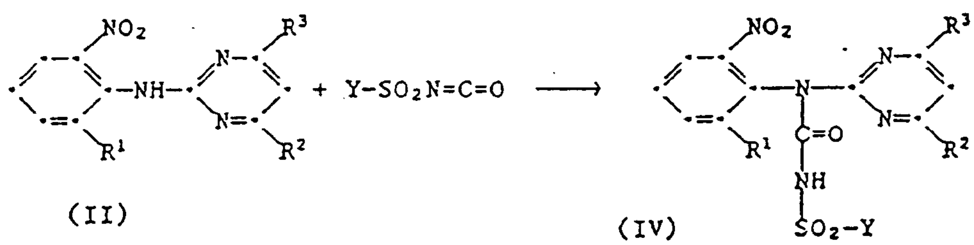

(II) (IV)

8. Agent herbicide contenant comme matière active un composé de formule I selon l'une des revendications 1 à 4, outre d'autres additifs et/ou supports.

9. Agent de régulation de la croissance des plantes contenant comme matière active un composé de formule I selon l'une des revendications 1 à 4, outre d'autres additifs et/ou supports.

10. Procédé de lutte contre la croissance végétale indésirable, caractérisé en ce qu'on fait agir une quantité à action herbicide d'un composé selon l'une des revendications 1 à 4, ou d'un agent selon la revendication 8, sur les plantes à combattre ou leur habitat.

11. Procédé pour influer sur la croissance des plantes, caractérisé en ce qu'on fait agir une quantité à action de régulation de la croissance des plantes d'un composé selon l'une des revendications 1 à 4 ou d'un agent selon la revendication 9 sur les plantes ou leur habitat.

**Revendications pour l'Etat contractant suivant : ES**

1. Procédé de préparation d'urées de formule I

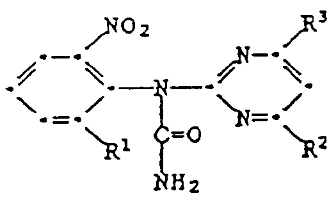

(I),

dans laquelle
$R^1$ représente un hydrogène, méthyle, fluor, chlore ou brome,
$R^2$ représente un halogènalcoyle en $C_{1-2}$ avec au moins 2 atomes de fluor et $R^4$ représente un alcoyle en $C_{1-4}$,
avec cette précision que $R^2$ ne représente pas un trifluorométhyle lorsque $R^3$ représente un méthyle, ainsi que les sels et composés d'addition désirés de formule I avec des acides, des bases ou des formateurs de complexes,
caractérisé en ce que l'on procède :
a) en faisant réagir une aniline de formule II avec du phosgène pour donner un chlorure de carbamine de formule III et en faisant réagir celui-ci dans une seconde étape avec $NH_3$ pour donner

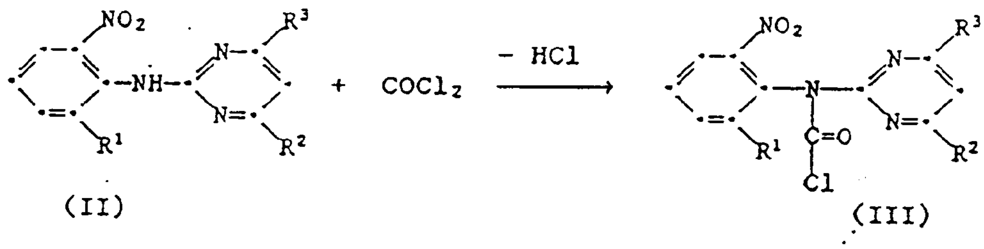


(II) (III)

- HCl

III + $NH_3$ → I

ou urée de formule I ou

b) en faisant réagir une aniline de formule II avec un isocyanate d'halogènesulfonyle pour donner une halogènesulfonylurée de formule IV

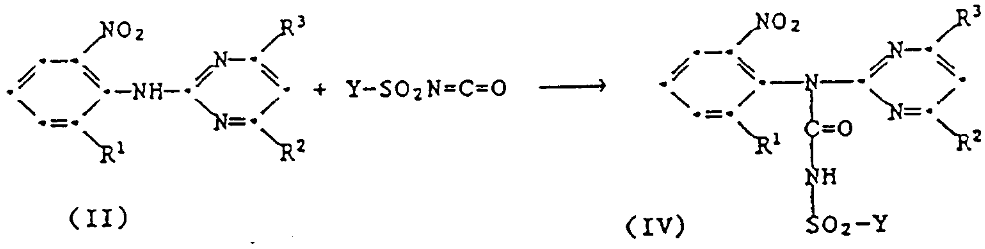


(II) (IV)

- HY

IV + $2H_2O$ → I

- $SO_4H_2$

et dans une seconde étape en hydrolysant directement celle-ci pour donner un composé de formule I, où Y représente un groupe séparable dans les conditions de la réaction, comme halogène, de préférence chlore, ou

c) en transposant une sulfonylurée de formule V sous l'action d'une base aqueuse pour donner une urée de formule I

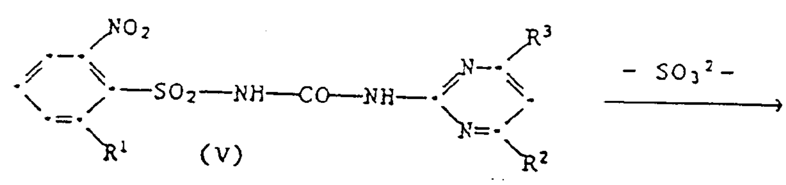


(V) (I),

où on utilise comme bases aqueuses de préférence NaOH/eau ou KOH/eau et le cas échéant on transforme le composé de formule I obtenu selon le procédé ci-dessus avec un acide, une base ou un formateur de complexe en un sel ou un produit d'addition.

**2.** Procédé selon la revendication 1 de préparation de 2-[N-carbamyl-N-(6-méthyl-2-nitrophényl)-amino]-4-trifluorométhyl-6-éthyl-pyrimidine ou de 2-[N-carbamyl-N-(2-nitrophényl)-amino]-4-trifluorométhyl-6-éthyl-pyrimidine.

**3.** Procédé de préparation d'urées de formule IV

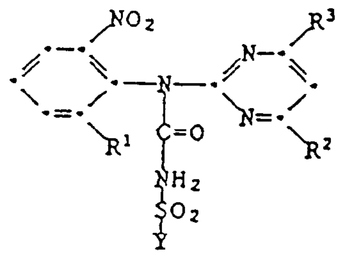

(IV),

où

Y représente un halogène,

$R^1$ représente un hydrogène, méthyle, fluor, chlore ou brome,

$R^2$ représente un halogènalcoyle en $C_{1-2}$ ayant au moins deux atomes de fluor,

$R^3$ représente un alcoyle en $C_{1-4}$,

avec cette précision que $R^2$ ne représente pas un trifluorométhyle lorsque $R^3$ représente un méthyle, caractérisé en ce qu'on fait réagir une aniline de formule II avec un isocyanate d'halogènesulfonyle

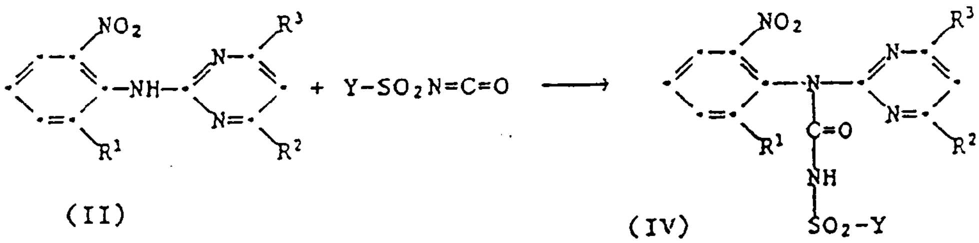


(II) (IV)

4. Procédé pour combattre la croissance végétale indésirable, caractérisé en ce qu'on fait agir une quantité à action herbicide d'un composé de formule V

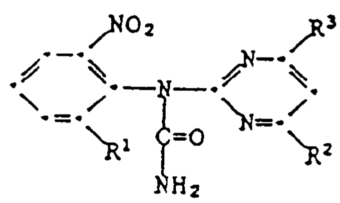

(I),

dans laquelle

$R^1$ représente un hydrogène, méthyle, fluor, chlore ou brome,

$R^2$ représente un halogènalcoyle en $C_{1-2}$ ayant au moins 2 atomes de fluor, et

$R^3$ représente un alcoyle en $C_{1-4}$,

avec cette précision que $R^2$ ne représente pas un trifluorométhyle lorsque $R^3$ représente un méthyle, ainsi que les sels et composés d'addition des urées de formule I avec des acides, des bases ou des formateurs de complexes, sur les plantes à combattre ou leur habitat.

5. Procédé pour influer sur la croissance végétale, caractérisé en ce qu'on fait agir une quantité à action de régulation de la croissance des plantes d'un composé de formule I

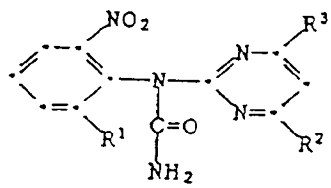

(I),

où

$R^1$ représente un hydrogène, méthyle, fluor, chlore ou brome,

$R^2$ représente un halogènalcoyle en $C_{1-2}$ ayant au moins 2 atomes de fluor,

$R^3$ représente un alcoyle en $C_{1-4}$,

avec cette précision que $R^2$ ne représente pas un trifluorométhyle lorsque $R^3$ représente un méthyle, ainsi que les sels et composés d'addition des urées de formule I avec des acides, des bases ou des formateurs de complexes, sur les plantes ou leur habitat.

**6.** Procédé de préparation d'un herbicide ou agent actif comme régulateur de la croissance des plantes, caractérisé en ce qu'on mélange un composé de formule I

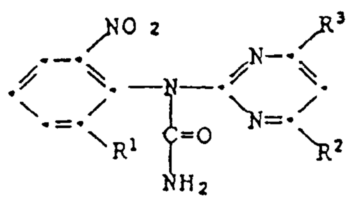

(I),

où

$R^1$ représente un hydrogène, méthyle, fluor, chlore ou brome,

$R^2$ représente un halogènalcoyle en $C_{1-2}$, ayant au moins 2 atomes de fluor et

$R^3$ représente un alcoyle en $C_{1-4}$,

avec cette précision que $R^2$ ne représente pas un trifluorométhyle lorsque $R^3$ représente un méthyle, ainsi que les sels et les composés d'addition désirés de formule I avec des acides, des bases ou des formateurs de complexes, avec les additifs et/ou supports habituels dans la technique de formulation.

**7.** Procédé pour combattre la croissance végétale indésirable ou pour influer sur la croissance végétale, caractérisé en ce qu'on fait agir une quantité à action herbicide ou à action de régulation de la croissance des plantes d'un agent selon la revendication 6 sur les plantes à combattre ou leur habitat.

**8.** Agent herbicide contenant un composé de formule I

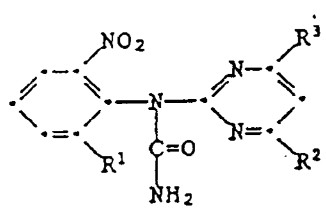

(I),

où

$R^1$ représente un hydrogène, méthyle, fluor, chlore ou brome,

$R^2$ représente un halogènalcoyle en $C_{1-2}$ ayant au moins 2 atomes de fluor, et

$R^3$ représente un alcoyle en $C_{1-4}$,

avec cette précision que $R^2$ ne représente pas un trifluorométhyle lorsque $R^3$ représente un méthyle,

caractérisé en ce qu'il contient de 0,1 à 80% de matière active de formule I, de 1 à 99,9% d'un additif solide ou liquide, et 0 à 25% d'un agent tensio-actif.